# FASCICULE DE BREVET EUROPEEN

(11) **EP 3 110 832 B1**
(45) Date de publication et mention de la délivrance du brevet: **15.07.2020**
(21) Numéro de dépôt: 15707320.6
(22) Date de dépôt: 24.02.2015
(51) Int. Cl.: C07K 14/415, C12N 15/82

(54) **PLANTES A RENDEMENT ACCRU ET METHODE D'OBTENTION DE TELLES PLANTES**
PFLANZEN MIT ERHÖHTER AUSBEUTE UND VERFAHREN ZUR HERSTELLUNG DER BESAGTEN PFLANZEN
PLANTS WITH INCREASED YIELD AND METHOD FOR PRODUCING SAID PLANTS

(30) Priorité: 24.02.2014 FR 1451445
(43) Date de publication de la demande: 04.01.2017
(60) Demande divisionnaire: 19215300.5
(73) Titulaire: Centre National de la Recherche Scientifique (CNRS), 75016 Paris (FR); Université Paris-Saclay, 91190 Saint-Aubin (FR)
(72) Inventeur: DE BONT, Linda, 91940 Les Ulis (FR); GAKIERE, Bertrand, 91190 Gif S/Yvette (FR)
(74) Mandataire: Regimbeau
(86) Numéro de dépôt international: PCT/EP2015/053850
(87) Numéro de publication internationale: WO 2015/124799

(56) Documents cités:
- EP-A1- 2 573 179
- WO-A1-99/04012
- WO-A1-2010/086220
- US-A1- 2007 016 976
- US-A1- 2013 310 585
- US-B1- 6 271 031
- A. P. MACHO ET AL: "ASPARTATE OXIDASE Plays an Important Role in Arabidopsis Stomatal Immunity", PLANT PHYSIOLOGY, vol. 159, no. 4, 1 août 2012 (2012-08-01), pages 1845-1856, XP055150776, ISSN: 0032-0889, DOI: 10.1104/pp.112.199810
- HUAWEN LIN ET AL: "Synthesizing and Salvaging NAD+: Lessons Learned from Chlamydomonas reinhardtii", PLOS GENETICS, vol. 6, no. 9, 9 septembre 2010 (2010-09-09), page e1001105, XP055085362, DOI: 10.1371/journal.pgen.1001105
- G. NOCTOR: "NAD(P) synthesis and pyridine nucleotide cycling in plants and their potential importance in stress conditions", JOURNAL OF EXPERIMENTAL BOTANY, vol. 57, no. 8, 19 mai 2006 (2006-05-19), pages 1603-1620, XP055150821, ISSN: 0022-0957, DOI: 10.1093/jxb/erj202

## Description

La présente invention concerne un procédé d'augmentation de la biomasse chez une plante, en particulier un procédé d'augmentation de la croissance d'une plante, plus particulièrement d'augmentation de la vitesse de croissance d'une plante, d'augmentation du rendement en grain, d'augmentation de la résistance au stress abiotique, la résistance au stress biotique et une augmentation de la vitesse de germination ainsi que les plantes qui en résultent, et ce via l'augmentation de l'expression de la L-aspartate oxydase au sein de la plante. Le procédé selon l'invention permet une augmentation des capacités photosynthétiques des plantes via une augmentation des quantités de NAD et de ses dérivés au sein desdites plantes.

Le développement d'une agriculture durable fait face à un défi majeur pour la communauté internationale dans les années à venir qui consiste à conserver une croissance de la production alimentaire au même rythme que celui de la population mondiale qui est malheureusement accompagnée d'une baisse mondiale de terres arables de grande qualité. Relever ce défi nécessitera des efforts dans plusieurs domaines, dont l'un sera de fournir des cultures d'une valeur nutritionnelle accrue, capables de résister aux divers stress environnementaux, de fournir plus de rendement avec moins d'intrants, de pousser plus vite, pour au final délivrer un meilleur rendement de culture et une biomasse plus importante.

La sécurité alimentaire a toujours été une priorité dans le monde entier et une préoccupation croissante pour l'impact environnemental de la production agricole qui nécessite le développement et l'utilisation de nouvelles méthodes pour améliorer la productivité tout en protégeant l'environnement. Il y a un besoin accru de meilleures approches pour améliorer le rendement des cultures dans des conditions de terrain différentes.

Afin d'augmenter les rendements et la biomasse disponible des plantes cultivées, des efforts ont été faits pour modifier le contenu de plantes en lignine. D'autres moyens pour augmenter la biomasse ont été étudiés tel que le génie génétique des plantes, tel que par exemple la manipulation génétique des régulateurs de croissance des végétaux ou des voies de photosynthèse.

WO 2012/041496 décrit une méthode de sélection par une technique de PCR ciblant un groupe de gènes marqueurs de l'efficacité d'utilisation énergétique afin d'obtenir des plantes ayant une meilleure vigueur et une meilleure tolérance aux stress abiotiques.

US 2006100573 décrit une méthode qui consiste à surproduire une enzyme du métabolisme secondaire C3'H impliquée dans la synthèse de lignine pour produire plus de paroi et donc plus de biomasse et plus de graines.

US 20060095981 décrit une méthode qui consiste à obtenir des plantes transgéniques contenant la voie bactérienne d'utilisation du glycolate afin de réduire les pertes de rendements liées à la photorespiration. Malheureusement ces plantes produisent de formes actives d'oxygène qui stressent les plantes produites (Kebeish *et al.,* 2007 ; Maier *et al.,* 2012).

Cependant chacune de ces méthodes vise une voie métabolique particulière qui ne permet pas de traiter les problèmes de production de biomasse, de résistance au stress abiotiques, de résistance au stress biotiques, de régularisation de la germination, de rendement en grain, de manière globale.

Il existe aussi une méthode de traitement de semence par une molécule insecticide de la famille des isonicotinoïdes à des fins d'augmentation de rendements (WO 01/26468). Cependant, l'utilisation de ces molécules, de structure proche des nucléotides à pyridine comme l'acide nicotinique et son précurseur le NAD⁺, est sujette à controverse en raison de l'impact que cela aurait sur la mortalité des abeilles.

US 6271031 décrit des polynucléotides encodant des polypeptides et entre autres la L-aspartate oxydase. L'objet de l'invention décrite dans US 6271031 vise cependant à modifier la production de quinolinate et n'enseigne aucune relation avec un phenotype lié à la croissance de la plante ainsi transformée. L'objet de l'invention décrit l'obtention d'ADNc codant la L-aspartate oxydase mais aucun exemple ne décrit les effets de son utilisation chez les plantes.

US2007/016976 décrit plusieurs milliers de séquences polynucléotidiques dont l'expression est altérée, soit vers une surexpression, soit vers une sous-expression, en réponse à une infection par un pathogène. Une sequence parmi celles-ci correspond à la L-aspartate oxydase mais rien n'est divulgué quant à un effet phenotypique sur la croissance d'une plante modifiée. En outre aucun exemple de plante dont l'expression de la L-aspartate oxydase n'est divulgué. Le fait que l'expression de milliers de gènes soit induite ou réprimée par l'altération métabolique conséquence d'une maladie ne prouve en rien l'implication de chacun des gènes dérégulés dans la résistance potentielle aux maladies.

WO9904012 décrit des procédés servant à augmenter la résistance de plantes à des pathogènes par expression d'une enzyme produisant une espèce d'oxygène réactive au peroxyde d'hydrogène ou d'une enzyme de dégradation d'oxalate. Ce document mentionne environ 25 enzymes, parmi lesquelle la L-aspartate oxydase, susceptibles de produire une espèce d'oxygène réactive. Cependant il est contesté que la L-aspartate oxydase puisse produire une espèce d'oxygène réactive et d'ailleurs aucun exemple de WO9904012 ne décrit cette production. En effet, à la différence de l'isoforme bactérienne de l'enzyme, la L-aspartate oxydase de plante ne semble pas capable de produire du péroxyde d'oxygène en présence d'oxygène moléculaire, mais son activité serait celle d'une succinate déshydrogénase. En outre, il est bien connu que chez les plantes, ce sont les NADPH oxydases qui sont les principales sources de production de formes actives d'oxygène en réponse à une attaque pathogène.

WO2010086220 décrit une méthode d'accroissement du rendement des plantes par modification du métabolisme azoté via transformation génétique, produisant des plantes plus vertes via une plus forte production de chlorophylle.. En ce qui concerne la modification d'expression de la L-aspartate oxydase, aucun exemple ne vient montrer l'effet d'une surexpression de la L-aspartate oxydase sur le phénotype des plantes. Aussi, aucun effet sur le rendement ou la vitesse de croissance de ces plantes n'est évoqué.

Les inventeurs de la présente invention ont maintenant découvert de façon surprenante que la surexpression de l'enzyme L-aspartate oxydase (première enzyme de la voie de biosynthèse du NAD⁺) au sein d'une plante conduit à une augmentation considérable des capacités photosynthétiques de la plante, ce qui se traduit par une accélération de la croissance, une augmentation de la biomasse produite, une augmentation du rendement, en particulier du rendement en grain. On observe aussi une accélération de la germination, une augmentation de la résistance au stress abiotique et une augmentation de la résistance au stress biotique chez les plantes surexprimant la L-aspartate oxydase.

La surexpression de la L-aspartate oxydase, première enzyme de la voie de biosynthèse du NAD dite *de novo* (Noctor *et al.,* 2006), a pour résultat d'augmenter les niveaux de NAD et de ses dérivés produits par les plantes. Il en résulte une augmentation importante des teneurs en NAD (pool de NAD représentant NAD⁺, NADH, NADP⁺ et NADPH) et de l'ensemble des nucléotides à pyridine dans les plantes surexprimant la L-aspartate oxydase, mais aussi du niveau d'autres métabolites énergétiques comme l'ATP. Sont ainsi obtenues des plantes, cellules végétales où toute partie de plantes au contenu énergétique élevé.

On observe ainsi des conséquences physiologiques remarquables comme une augmentation des capacités photosynthétiques et des vitesses de croissance des plantes surexprimant la L-aspartate oxydase. Il en résulte une augmentation significative de biomasse, mais aussi des rendements en graines lorsque l'on cultive les plantes surexprimant la L-aspartate oxydase à maturité. Ces augmentations de rendements sont fortement corrélées aux niveaux de surproduction de la L-aspartate oxydase et de NAD.

Une très forte augmentation de la résistance des plantes à des conditions environnementales de stress abiotique comme une combinaison de forte chaleur et/ou forte lumière est observée chez les plantes surexprimant la L-aspartate oxydase.

Cette observation permet d'envisager une diminution des interventions phytosanitaires sur des cultures de plantes surproduisant la L-aspartate oxydase et aussi d'étendre la culture d'une espèce surproduisant la L-aspartate oxydase au-delà de la zone géographique habituellement réservée à l'espèce cultivée ne surexprimant pas la L-aspartate oxydase.

Une augmentation significative de la résistance des plantes à des conditions environnementales de stress biotique comme une attaque de pucerons est observée chez des plantes surexprimant la L-aspartate oxydase.

Cette observation permet d'envisager une diminution des traitements phytosanitaires, notamment insecticides, sur des cultures de plantes surproduisant la L-aspartate oxydase, particulièrement lorsque les conditions environnementales sont propices à la multiplication des pathogènes ou ravageurs.

Les graines des plantes surexprimant la L-aspartate oxydase germent plus vite et de manière plus homogène que celles de plantes ne surexprimant pas la L-asparate-oxydase. Cela peut permettre à la culture de plantes surproduisant la L-aspartate oxydase de s'installer plus vite, limitant la concurrence d'espèces advantices et les pertes de rendements. Pour cette raison aussi, des cultures de plantes surexprimant la L-asparatate oxydase auront des besoins moindres en traitements herbicides ciblant les advantices des cultures.

Dans des conditions environnementales défavorables telles qu'un milieu carencé en azote, les graines de plantes surexprimant la L-aspartate oxydase germent plus vite et, très important, maintiennent une capacité germinative maximale dans ces conditions, alors que la capacité germinative des plantes ne surexprimant pas la L-aspartate oxydase est beaucoup plus faible. La présente invention permet d'envisager une diminution de l'apport d'intrants azotés sur des cultures surexprimant la L-aspartate oxydase.

La surexpression de la L-aspartate oxydase chez des plantes selon l'invention permet de s'affranchir d'un traitement de priming des graines, en particulier chez les espèces potagères, méthodologie financièrement coûteuse habituellement utilisée pour lever la dormance, accélérer et homogénéiser la germination des semences commerciales.

La présente invention concerne donc une méthode pour améliorer au moins un caractère phénotypique choisi parmi le rendement en grains, la résistance au stress abiotique, la résistance au stress biotique, la vitesse de germination ou la croissance, chez une plante, ladite méthode comprenant la surexpression de la L-aspartate oxydase ; ladite méthode comprend la transformation d'une cellule végétale avec au moins un acide nucléique qui code pour la L-aspartate oxydase, et la génération à partir d'une telle cellule, d'une plante qui surexprime la L-aspartate oxydase.

Dans un mode particulier, la méthode selon l'invention est une méthode pour améliorer le le rendement en graines, chez une plante, laquelle méthode comprend la transformation d'une cellule végétale avec au moins un acide nucléique qui code pour la L-aspartate oxydase, générant ainsi une plante qui surexprime la L-aspartate oxydase, et la culture de la plante à maturité.

Dans un mode particulier, la méthode selon l'invention est une méthode pour améliorer la vitesse de germination chez une plante, laquelle méthode comprend la transformation d'une cellule végétale avec au moins un acide nucléique qui code pour la L-aspartate oxydase, générant ainsi une plante qui surexprime la L-aspartate oxydase, et la culture de la plante à maturité.

La présente description divulgue une méthode pour améliorer au moins un caractère phénotypique choisi parmi la biomasse, le rendement, en particulier le rendement en grains, la résistance au stress abiotique, la résistance au stress biotique, la vitesse de germination ou la croissance, chez une plante, ladite méthode comprenant la surexpression de la L-aspartate oxydase, qui se traduit par une augmentation des quantités de NAD et de ses dérivés.

Dans un autre mode de réalisation, la méthode selon la présente description vise à améliorer la production de biomasse chez une plante, laquelle méthode comprend la transformation d'une cellule végétale avec au moins un acide nucléique qui code pour la L-aspartate oxydase, générant ainsi une plante qui surexprime la L-aspartate oxydase, et la culture de la plante à maturité.

Les plantes selon l'invention présentent ainsi une augmentation des quantités de NAD et de ses dérivés.

Les méthodes selon l'invention visent aussi une augmentation des quantités de NAD et de ses dérivés.

C'est aussi un objet de la présente invention que de fournir une méthode pour améliorer la résistance au stress abiotique et/ou la résistance au stress biotique chez une plante, laquelle méthode comprend la transformation d'une cellule végétale avec au moins un acide nucléique qui code pour la L-aspartate oxydase, générant ainsi une plante qui surexprime la L-aspartate oxydase, et la culture de la plante à maturité.

Dans une méthode selon la présente invention le au moins un acide nucléique qui code pour la L-aspartate oxydase est sous le contrôle d'un promoteur assurant la surexpression de la L-aspartate oxydase.

Dans un autre mode préférentiel de réalisation de la présente invention le au moins un acide nucléique comprend un acide nucléique choisi dans le groupe constitué par SEQ ID N°1, SEQ ID N°3, SEQ ID N°4, SEQ ID N°5, SEQ ID N°6, SEQ ID N°7, SEQ ID N°8, SEQ ID N°9, SEQ ID N°10, SEQ ID N°11, SEQ ID N°12, SEQ ID N°13, SEQ ID N°14, SEQ ID N°15, SEQ ID N°16.

Dans un mode de réalisation de la méthode selon l'invention le au moins un acide nucléique comprend un acide nucléique selon SEQ ID N°1.

Une méthode selon la présente invention vise aussi une méthode dans laquelle, la plante est choisie dans le groupe constitué par blé, orge, riz, maïs, sorgho, tournesol, colza, soja, coton, pois, haricot, manioc, mangue, banane, pomme de terre, tomate, poivron, melon, courgette, pastèque, laitue, choux, aubergine, peuplier.

Dans une méthode particulière de réalisation selon la présente invention, la plante est le riz (*Oryza sativa),* le blé, l'orge ou le maïs.

Selon un mode divulgué dans la présente description, la surexpression de la L-aspartate oxydase est réalisée par introgression d'un élément génétique codant pour une surexpression de la L-aspartate oxydase.
Les plantes décrites ici présentent ainsi une augmentation des quantités de NAD et de ses dérivés.

Les méthodes selon l'invention visent aussi une augmentation des quantités de NAD et de ses dérivés.

La présente description divulgue une méthode dans laquelle l'introgression d'un élément génétique codant pour une surexpression de la L-aspartate oxydase est obtenue par fusion de protoplastes.

La présente description divulgue une méthode dans laquelle l'introgression d'un élément génétique codant pour une surexpression de la L-aspartate oxydase est obtenue par sauvetage d'embryon.

La présente description divulgue une méthode pour la production de plante présentant au moins un caractère phénotypique amélioré choisi parmi la biomasse, la vitesse de germination, le rendement, en particulier le rendement en grains, la résistance au stress abiotique, la résistance au stress biotique, la vitesse de germination, la croissance, méthode comprenant la détection de la présence d'un élément génétique, en particulier une séquence d'acide nucléique, lié à la surexpression de la L-aspartate oxydase dans une plante donneuse et le transfert de l'élément génétique, en particulier une séquence d'acide nucléique, lié à la surexpression de la L-aspartate oxydase ainsi détecté, depuis la plante donneuse vers une plante receveuse.

La surexpression de la L-aspartate oxydase se traduit par une augmentation des quantités de NAD et de ses dérivés.

Dans un mode de réalisation particulier la détection est réalisée à l'aide d'au moins un marqueur moléculaire.

Dans une forme alternative, la détection est réalisée par la mesure de l'activité enzymatique de la L-aspartate oxydase dans la plante donneuse.

La présente description divulgue une méthode pour augmenter au moins un caractère phénotypique choisi parmi la biomasse, la vitesse de germination, le rendement, en particulier le rendement en grains, la résistance au stress abiotique, la résistance au stress biotique, la vitesse de germination, la croissance, chez une plante comprenant la fourniture d'une population de plantes et la sélection des individus de la population qui présentent une expression de la L-aspartate oxydase la plus élevée possible.

Dans un mode de réalisation la population de plantes est une population de plantes mutantes.

De manière particulière, la population de plantes mutantes est obtenue par TILLING.

La présente description divulgue une méthode qui comprend la sélection, au sein d'une population de plantes, d'au moins une plante présentant une surexpression de la L-aspartate oxydase par rapport à l'expression de la L-aspartate oxydase des plantes parents.

La présente description divulgue une méthode dans laquelle l'introgression comprend :
a) La fourniture d'une plante présentant un niveau d'expression donné de la L-aspartate oxydase,
b) La fourniture d'une plante présentant un niveau d'expression accru de la L-aspartate oxydase par rapport à la plante fournie en a),
c) Le croisement de la plante fournie en a) avec la plante fournie en b),
d) La génération d'une progéniture issue du croisement c),
e) La sélection au sein de la progéniture d'au moins une plante présentant un niveau d'expression de la L-aspartate oxydase supérieur à celui de la plante fournie en b).

Dans un mode particulier, la méthode décrite ci avant comprend une étape supplémentaire de croisement de la plante sélectionnée en e) avec la plante fournie en b) suivie d'une étape supplémentaire de sélection au sein de la progéniture obtenue d'au moins une plante présentant un niveau d'expression de la L-aspartate oxydase supérieur à celui de la plante sélectionnée en e).

La présente description divulgue une méthode de sélection de plante, caractérisée en ce qu'elle comprend la recherche d'un allèle du gène de l'enzyme L-aspartate oxydase possédant une mutation résultant en une amélioration d'au moins un caractère phénotypique choisi parmi la biomasse, le rendement en grains, la résistance au stress abiotique, la résistance au stress biotique ou, la vitesse de croissance ou la vitesse de germination.

La présente description divulgue une plante susceptible d'être obtenue par une méthode selon l'un des modes de réalisation décrit ici.

Enfin, La présente description divulgue l'utilisation d'une plante telle qu'obtenue par une méthode selon l'un des modes de réalisation décrit ici, ou d'un dérivé d'une telle plante, pour la préparation d'une composition destinée à l'alimentation humaine, l'alimentation animale ou la préparation de biocarburants.

La présente description divulgue l'utilisation d'au moins un acide nucléique qui code pour la L-aspartate oxydase, et de la protéine codée L-aspartate oxydase, pour l'obtention d'une plante qui présente au moins un caractère phénotypique choisi parmi une biomasse accrue, une vitesse de germination accrue, une vitesse de croissance accrue, un rendement accru, en particulier un rendement en grains accru, une résistance au stress abiotique accrue, une résistance au stress biotique accrue, une vitesse de germination accrue, une croissance accélérée, via la surexpression de la L-aspartate oxydase.

La présente description divulgue un procédé d'obtention d'au moins un caractère phénotypique choisi parmi une biomasse accrue, une vitesse de germination accrue, un rendement accru, en particulier un rendement en grains accru, une résistance au stress abiotique accrue, une résistance au stress biotique accrue, une vitesse de germination accrue, une croissance accélérée, chez une plante, comprenant l'étape consistant à surexprimer la L-aspartate oxydase dans des cellules de ladite plante.

La surexpression de la L-aspartate oxydase dans les cellules de la plante peut être obtenue par divers moyens à la disposition de l'homme du métier, que ce soit par transgénèse ou par transformation.

La surexpression de la L-aspartate oxydase se traduit par une augmentation des quantités de NAD et de ses dérivés.

La présente description divulgue un procédé de production d'une plante ou d'une partie d'une plante, présentant au moins un caractère phénotypique choisi parmi une biomasse accrue, une vitesse de germination accrue, un rendement accru, en particulier un rendement en grains accru, une résistance au stress abiotique accrue, une résistance au stress biotique accrue, une vitesse de germination accrue, une croissance accélérée, lequel procédé comprend la transformation d'une cellule végétale avec au moins un acide nucléique qui code pour la L-aspartate oxydase, et générer à partir d'une telle cellule, une plante qui surexprime la L-aspartate oxydase.

La méthode selon l'invention pour améliorer au moins un caractère phénotypique choisi parmi le rendement en grains, la résistance au stress abiotique, la résistance au stress biotique ou la vitesse de germination ou la croissance chez une plante, comprend la transformation d'une cellule végétale avec au moins un acide nucléique qui code pour la L-aspartate oxydase, et la génération à partir d'une telle cellule, d'une plante qui surexprime la L-aspartate oxydase.

Dans un mode de réalisation particulier, le au moins acide nucléique qui code pour la L-aspartate oxydase est sous le contrôle d'un promoteur assurant la surexpression de la L-aspartate oxydase.

La présente description divulgue un procédé de production d'une cellule végétale, une plante ou partie de plante, présentant au moins un caractère phénotypique choisi parmi une biomasse accrue, une vitesse de germination accrue, un rendement accru, en particulier un rendement en grains accru, une résistance au stress abiotique accrue, une résistance au stress biotique accrue, une vitesse de germination accrue, une croissance accélérée, lequel procédé comprend l'étape consistant à transformer une cellule végétale avec au moins un acide nucléique qui code pour la L-aspartate oxydase, et peut comprendre l'étape supplémentaire de génération d'une plante à partir de celle-ci qui surexprime la L-aspartate oxydase.

La présente description divulgue un procédé pour obtenir des plantes enrichies en nucléotides à pyridine, à biomasse et rendement en graines augmentés et présentant une forte résistance à des stress environnementaux. L'augmentation ciblée des teneurs en NAD via la surexpression de la L-aspartate oxydase au sein des cellules de la plante conduit à une augmentation des teneurs en métabolites énergétiques. Cette stratégie de surexpression d'une séquence génétique représente une nouvelle amélioration génétique qui permet aux plantes de dépasser leur potentiel maximal de rendement.

La méthode la présente invention procure les avantages suivants :
- augmenter de façon constitutive la biomasse des plantes tout au long de leur développement, et donc d'accroître le rendement des cultures;
- augmenter significativement le rendement en graine des plantes;
- accroître la précocité de production de biomasse, de graine et la précocité germinative;
- permettre de produire des plantes résistantes aux conditions de fortes chaleur et lumière, ce qui limite les pertes de rendement dans ces conditions ;
- permettre aux plantes de mieux résister à divers stress biotiques comme les attaques par des ravageurs tels que des pucerons, ce qui permet d'envisager une réduction des traitements phytosanitaires des cultures, insecticides notamment ;
- permettre aux plantes d'augmenter leur vitesse de germination, donc d'installation d'une culture ;
- permettre aux plantes une meilleure levée de dormance (particulièrement vrai chez certaines espèces végétales qui nécessitent un priming pour germer) ;
- permettre aux plantes de maintenir une forte capacité germinative, particulièrement en milieu pauvre en azote, ce qui permet d'envisager une limitation des apports azotés sur ces cultures, donc de limiter les coûts et la pollution agricole ;
- et permettre de produire des plantes au meilleur statut énergétique, ce qui permet d'envisager une réduction des traitements phytosanitaires des cultures.

En outre, la méthode mise au point pour mesurer l'activité de la L-aspartate oxydase à partir de tissus de la plante peut être utilisée comme un marqueur biochimique de l'état de l'homéostasie énergétique pour l'amélioration des plantes mais aussi comme marqueur de sélection de plantes selon l'invention, surexprimant la L-aspartate oxydase.

Les parties de la plante peuvent être des racines, des feuilles, le tronc, la tige, les fruits, les organes de réserve et les fleurs par exemple.

L'invention propose en outre une méthode pour améliorer le rendement en graines, chez une plante, laquelle méthode comprend la transformation d'une cellule végétale avec au moins un acide nucléique qui code pour la L-aspartate oxydase, générant ainsi une plante qui surexprime la L-aspartate oxydase, et la culture de la plante à maturité.

La présente description divulgue une méthode pour améliorer la production de biomasse chez une plante, laquelle méthode comprend la transformation d'une cellule végétale avec au moins un acide nucléique qui code pour la L-aspartate oxydase, générant ainsi une plante qui surexprime la L-aspartate oxydase, et la culture de la plante à maturité.

L'invention propose en outre une méthode pour améliorer la résistance au stress abiotique chez une plante, laquelle méthode comprend la transformation d'une cellule végétale avec au moins un acide nucléique qui code pour la L-aspartate oxydase, générant ainsi une plante qui surexprime la L-aspartate oxydase, et la culture de la plante à maturité.

L'invention propose en outre une méthode pour améliorer la résistance au stress biotique chez une plante, laquelle méthode comprend la transformation d'une cellule végétale avec au moins un acide nucléique qui code pour la L-aspartate oxydase, générant ainsi une plante qui surexprime la L-aspartate oxydase, et la culture de la plante à maturité.

Dans une mode de réalisation particulier de la présente invention, la transformation d'une cellule végétale comprend la transformation avec au moins un acide nucléique qui code pour la L-aspartate oxydase. Ainsi dans un tel cas de figure, la transformation comprend la transformation avec un acide nucléique codant pour la L-aspartate oxydase en multi-copies, ce qui permet une production de la protéine en quantité accrue, et donc une surexpression de cette enzyme. La transformation peut être réalisée avec 2 copies d'un acide nucléique codant pour la L-aspartate oxydase, particulièrement avec 3 copies d'un acide nucléique codant pour la L-aspartate oxydase, particulièrement encore avec 4 copies d'un acide nucléique codant pour la L-aspartate oxydase, plus particulièrement encore avec 5 copies, ou plus, de l'acide nucléique codant pour la L-aspartate oxydase.

Dans un mode de réalisation particulier d'une méthode selon l'invention, l'acide nucléique qui code pour la L-aspartate oxydase est sous le contrôle d'un promoteur assurant la surexpression de la L-aspartate oxydase.

La présente description divulgue une cellule végétale, une plante ou partie de plante, qui est transgénique pour au moins un acide nucléique qui code pour la L-aspartate oxydase et qu'il surexprime.
Un acide nucléique codant pour la L-aspartate oxydase peut être n'importe quel acide nucléique encodant l'enzyme fonctionnelle de telle manière que lorsqu'introduit dans une cellule hôte et sous le contrôle d'un promoteur adéquat, la quantité de L-aspartate oxydase et/ou son activité enzymatique au sein de la cellule sont augmentées.

A titre d'exemple, un acide nucléique codant pour la L-aspartate oxydase peut être l'acide nucléique selon la séquence SEQ ID N°1 correspondant à *Arabidopsis thaliana.*

Cette séquence SEQ ID N°1 correspond à un acide nucléique codant pour la L-aspartate oxydase *d'Arabidopsis thaliana* à proprement parler et comprend une portion codant pour un peptide d'adressage plastidial.

Plus particulièrement, l'acide nucléique codant pour la L-aspartate oxydase :
i) a une séquence présentant au moins 55 % d'homologie avec la SEQ ID N°1 ou sa séquence complémentaire, ou
ii) a une séquence qui s'hybride à la SEQ ID N°1 ou sa séquence complémentaire, dans des conditions de stringence, et qui code pour la L-aspartate oxydase.

Dans un mode de réalisation particulier, l'acide nucléique codant pour la L-aspartate oxydase a une séquence présentant au moins 60%, au moins 65%, au moins 70 %, au moins 75%, au moins 80 %, au moins 85%, au moins 90 %, au moins 92%, au moins 93%, au moins 94%, au moins 95%, au moins 96%, au moins 96%, au moins 97%, au moins 98%, au moins 99% d'homologie avec la SEQ ID N°1.

L'acide nucléique codant pour la L-aspartate oxydase à transférer afin de surexprimer la L-aspartate oxydase peut également être un acide nucléique homologue de la plante hôte choisi parmi les acides nucléique de plantes, de microorganismes ou d'algues.

Dans le cadre de la mise en œuvre d'acide nucléique de microorganismes codant pour la L-aspartate oxydase, il conviendra de réaliser une construction par ligation avec une séquence codant pour un peptide d'adressage tel que, par exemple un peptide d'adressage plastidial, tel que celui de la petite sous-unité de la rubisco.

Le terme «homologue» ou « homologie » se rapporte à tout acide nucléique, ou protéine, ayant une ou plusieurs modification(s) de séquence par rapport à tout ou partie de la séquence de SEQ ID N°1 ou de la séquence SEQ ID N°2, respectivement, tout en conservant la plupart ou la totalité de l'activité de la L-aspartate oxydase.

Dans un mode de réalisation de la présente invention, la surexpression de la L-aspartate oxydase dans les plantes peut être obtenue en utilisant des acides nucléiques ou des protéines, comprenant l'une quelconque des séquences de nucléotides ou d'acides aminés suivants : SEQ ID N°1, SEQ ID N°2, SEQ ID N°3, SEQ ID N°4, SEQ ID N°5, SEQ ID N°6, SEQ ID N°7, SEQ ID N°8, SEQ ID N°9, SEQ ID N°10, SEQ ID N°11, SEQ ID N°12, SEQ ID N°13, SEQ ID N°14, SEQ ID N°15, SEQ ID N°16.

Dans un mode de réalisation particulier de la présente invention, l'acide nucléique codant la L-aspartate oxydase comprend un acide nucléique choisi dans le groupe constitué par SEQ ID N°1, SEQ ID N°3, SEQ ID N°4, SEQ ID N°5, SEQ ID N°6, SEQ ID N°7, SEQ ID N°8, SEQ ID N°9, SEQ ID N°10, SEQ ID N°11, SEQ ID N°12, SEQ ID N°13, SEQ ID N°14, SEQ ID N°15, SEQ ID N°16.

Dans un mode de réalisation particulier, l'acide nucléique codant pour la L-aspartate oxydase a une séquence présentant au moins 60%, au moins 65%, au moins 70 %, au moins 75%, au moins 80 %, au moins 85%, au moins 90 %, au moins 92%, au moins 93%, au moins 94%, au moins 95%, au moins 96%, au moins 96%, au moins 97%, au moins 98%, au moins 99% d'homologie avec la séquence d'un acide nucléique choisi dans le groupe constitué par SEQ ID N°1, SEQ ID N°3, SEQ ID N°4, SEQ ID N°5, SEQ ID N°6, SEQ ID N°7, SEQ ID N°8, SEQ ID N°9, SEQ ID N°10, SEQ ID N°11, SEQ ID N°12, SEQ ID N°13, SEQ ID N°14, SEQ ID N°15, SEQ ID N°16.

La présente description divulgue une cassette d'expression comprenant un promoteur exprimable dans une plante fonctionnellement lié à une région codante contenant au moins un acide nucléique codant pour une L-aspartate oxydase, dans lequel ledit promoteur n'est pas un promoteur de L-aspartate oxydase. Ledit promoteur peut être un promoteur 35S, un promoteur d'ubiquitine ou un promoteur d'actine, par exemple.

Le terme «transgénique» signifie que la cellule végétale ou de la plante comprend dans son génome au moins un acide nucléique codant pour la L-aspartate oxydase qui est étranger à cette plante ou cellule de plante, ou qui comprend dans son génome au moins une séquence codante endogène de la L-aspartate oxydase fonctionnellement liée à au moins une région de régulation, par exemple, un promoteur, qui n'est pas présent dans le gène endogène de cette plante ou cellule végétale. En général, l'acide nucléique étranger est intégré de manière stable dans le génome de telle sorte que le polynucléotide est transmis aux générations successives. Le terme transgénique inclut aussi le cas dans lequel la cellule végétale ou la plante comprend dans son génome deux ou plus de deux acides nucléiques endogènes ou exogènes de l'espèce de la cellule ou de la plante codant pour la L-aspartate oxydase ; permettant ainsi une surexpression de la L-asparate-oxydase. Le terme «surexpression» est ici destiné à signifier à la fois une augmentation de la quantité de la L-aspartate oxydase par rapport à la quantité exprimée dans une plante contrôle, et une expression ectopique de cette enzyme, dans un tissu ou un compartiment et/ou à un stade de développement où il n'est normalement pas exprimé. Elle englobe la situation dans laquelle la L-aspartate oxydase est endogène ou hétérologue, c'est à dire quand il s'agit d'un organisme, tel qu'une plante, différente de la cellule hôte, ou dans laquelle au moins une région de régulation de transcription de la L-aspartate oxydase n'est pas présente dans le gène endogène. La quantité et/ou l'activité de la protéine L-aspartate oxydase exprimée dans une cellule végétale peut être déterminée en nmoles d'iminoaspatate produites/min/mg de protéine ou en nmoles d'iminoaspartate produites/min/mg de chlorophylle, par mesure du NH₄⁺ relargué par la décomposition quasi instantanée de l'iminoaspartate dans les conditions de mesure de l'essai.

Le terme «surexpression» signifie aussi que l'activité enzymatique de la L-asparte-oxydase produite au sein de la cellule hôte après introduction de la séquence codant pour la L-aspartate oxydase, à quantité égale d'enzyme produite, est supérieure à l'activité enzymatique de la L-aspartate oxydase de la cellule hôte avant l'introduction de ladite séquence. Une telle suractivité spécifique peut être due à une différence de structure primaire, secondaire ou tertiaire de la protéine due à une différence dans la séquence nucléique l'encodant.

La surexpression de la L-aspartate oxydase se traduit par une augmentation des quantités de NAD et de ses dérivés.

Les plantes ici décrites présentent ainsi une augmentation des quantités de NAD et de ses dérivés.

La méthode permet aussi une augmentation des quantités de NAD et de ses dérivés.

Le NAD (nicotinamide adénine dinucléotide) est une coenzyme d'oxydoréduction présent dans toutes les cellules vivantes. Le composé est un dinucléotide, puisqu'il est composé de deux nucléotides liés par leurs groupes phosphate. Un des nucléotides contient une adénine tandis que l'autre contient un nicotinamide. Dans le métabolisme, le NAD⁺ est impliqué dans les réactions redox en transportant des électrons. Cette coenzyme est présente sous deux formes dans la cellule. NAD⁺ est un agent d'oxydation et NADH est un agent de réduction.

L'expression NAD et ses dérivés s'entend du NAD, du NAD+, du NADP, NADP+ et NADPH.

Le nicotinamide adénine dinucléotide phosphate (NADP) est un coenzyme d'oxydoréduction. Il est très proche du nicotinamide adénine dinucléotide (NAD), dont il diffère par la présence d'un groupement phosphate sur le second carbone du β-D-ribofurannose du résidu adénosine. Sa forme réduite est désignée par NADPH ou NADPH2 ou encore NADPH+H⁺.

Une «augmentation» d'au moins un caractère phénotypique choisi parmi la croissance, la taille et/ou poids, le rendement, en particulier le rendement en graines, la vitesse de croissance, la croissance, la vitesse de germination, la biomasse, la résistance au stress abiotique, la résistance au stress biotique, observée chez les plantes selon l'invention qui surexpriment la L-aspartate oxydase indique que ce au moins un caractère est quantitativement significativement supérieur à celui des plantes contrôles de la même espèce qui n'ont pas subi de transformation avec un acide nucléique codant pour la L-aspartate oxydase, ou qui n'ont pas subi d'introgression d'un élément génétique codant pour ladite L-aspartate oxydase, lorsqu'elles sont cultivées dans les mêmes conditions de croissance .

Par l'expression «plante contrôle», dans le cadre de l'invention, on entend une plante qui a le même fond génétique qu'une plante selon la présente invention dans lequel la plante contrôle ne dispose pas de l'acide nucléique ou de l'élément génétique permettant une surexpression de la L-aspartate oxydase selon la présente invention ; surexpression liée à une augmentation d'un caractère phénotypique choisi parmi une biomasse accrue, une vitesse de germination accrue, un rendement accru, en particulier un rendement en grains accru, une résistance au stress abiotique accrue, une résistance au stress biotique accrue, une vitesse de germination accrue, une croissance accélérée. Une plante contrôle est cultivée pour la même durée de temps et dans les mêmes conditions qu'une plante selon la présente invention.

L'expression «variété», «cultivar» ou «obtention végétale» est ici entendue selon la définition de l'UPOV. Ainsi, une plante contrôle peut être une variété, lignée pure ou un hybride, à condition d'avoir le même fond génétique que la plante selon la présente invention à l'exception de l'acide nucléique, ou de l'élément génétique, permettant l'amélioration d'au moins un caractère phénotypique choisi parmi la biomasse, la vitesse de germination, le rendement, en particulier le rendement en grains, la résistance au stress abiotique, la résistance au stress biotique, la vitesse de germination, la croissance, selon de la présente invention et liée à la surexpression de la L-aspartate oxydase.

Dans les méthodes ici décrites, l'acide nucléique ou l'élément génétique codant pour la L-aspartate oxydase peut être hétérologue par rapport à la plante dans laquelle il est introduit ou peut appartenir à la même espèce et ce dans la mesure où il peut être exprimé dans les plantes en des quantités supérieures à la quantité classiquement produite par une plante non transformée ou une plante qui ne contenait pas la séquence ou l'élément introduit ou introgressé. Ainsi, toute séquence nucléotidique codant pour la L-aspartate oxydase peut être utilisée, par exemple, une séquence de type sauvage pour le gène de la L-aspartate oxydase, une séquence mutée au niveau de la portion codante de l'enzyme entraînant une activité spécifique plus élevée ou une séquence mutée au niveau de la région promotrice entraînant une plus forte production de la protéine et donc une surexpression de l'enzyme, voire une combinaison des deux cas de figure.

Avantageusement, un acide nucléique codant pour la L-aspartate oxydase utilisé pour transformer les cellules ou les plantes selon l'invention comprend un acide nucléique codant pour la protéine de la SEQ ID N°2, par exemple la séquence codante de l'ADNc *d'Arabidopsis thaliana* de la SEQ ID N°1.

Dans un mode de réalisation préféré de la méthode selon l'invention, la plante est choisie dans le groupe constitué par blé, orge, riz, maïs, sorgho, tournesol, colza, soja, coton, pois, haricot, manioc, mangue, banane, pomme de terre, tomate, poivron, melon, courgette, pastèque, laitue, choux, aubergine, peuplier.

Dans un mode de réalisation préféré de la méthode selon l'invention, la plante est choisie dans le groupe comprenant blé, orge, riz, maïs.
Dans un mode encore préféré de réalisation de la méthode selon l'invention, la plante est le riz asiatique (*Oryza sativa)* ou le riz africain (*Oryza glaberrima*) ou le riz hybride de ces deux espèces.

La présente description divulgue la surexpression de la L-aspartate oxydase dans une monocotylédone, en particulier le riz (*Oryza sativa),* en surexprimant la L-aspartate oxydase codée par un acide nucléique choisi dans le groupe constitué par SEQ ID N°1, SEQ ID N°3, SEQ ID N°4, SEQ ID N°5, SEQ ID N°6, SEQ ID N°7, SEQ ID N°8, SEQ ID N°9, SEQ ID N°10, SEQ ID N°11, SEQ ID N°12, SEQ ID N°13, SEQ ID N°14, SEQ ID N°15, SEQ ID N°16 dans des cellules de plantes de riz.

La présente description divulgue la surexpression de la L-aspartate oxydase dans une monocotylédone, en particulier le riz (*Oryza glaberrima*), en surexprimant la L-aspartate oxydase codée par un acide nucléique choisi dans le groupe constitué par SEQ ID N°1, SEQ ID N°3, SEQ ID N°4, SEQ ID N°5, SEQ ID N°6, SEQ ID N°7, SEQ ID N°8, SEQ ID N°9, SEQ ID N°10, SEQ ID N°11, SEQ ID N°12, SEQ ID N°13, SEQ ID N°14, SEQ ID N°15, SEQ ID N°16 dans des cellules de plantes de riz.

La présente description divulgue la surexpression de la L-aspartate oxydase dans une monocotylédone, en particulier le blé (*Triticum*), en surexprimant la L-aspartate oxydase codée par un acide nucléique choisi dans le groupe constitué par SEQ ID N°1, SEQ ID N°3, SEQ ID N°4, SEQ ID N°5, SEQ ID N°6, SEQ ID N°7, SEQ ID N°8, SEQ ID N°9, SEQ ID N°10, SEQ ID N°11, SEQ ID N°12, SEQ ID N°13, SEQ ID N°14, SEQ ID N°15, SEQ ID N°16 dans des cellules de plantes de blé.

La présente description divulgue la surexpression de la L-aspartate oxydase dans une monocotylédone, en particulier l'orge (*Hordeum vulgare*), en surexprimant la L-aspartate oxydase codée par un acide nucléique choisi dans le groupe constitué par SEQ ID N°1, SEQ ID N°3, SEQ ID N°4, SEQ ID N°5, SEQ ID N°6, SEQ ID N°7, SEQ ID N°8, SEQ ID N°9, SEQ ID N°10, SEQ ID N°11, SEQ ID N°12, SEQ ID N°13, SEQ ID N°14, SEQ ID N°15, SEQ ID N°16 dans des cellules de plantes d'orge.

La présente description divulgue la surexpression de la L-aspartate oxydase dans une monocotylédone, en particulier le maïs (*Zea mays*), en surexprimant la L-aspartate oxydase codée par un acide nucléique choisi dans le groupe constitué par SEQ ID N°1, SEQ ID N°3, SEQ ID N°4, SEQ ID N°5, SEQ ID N°6, SEQ ID N°7, SEQ ID N°8, SEQ ID N°9, SEQ ID N°10, SEQ ID N°11, SEQ ID N°12, SEQ ID N°13, SEQ ID N°14, SEQ ID N°15, SEQ ID N°16 dans des cellules de plantes de maïs.

La présente description divulgue la surexpression de la L-aspartate oxydase dans une monocotylédone, en particulier le sorgho (*Sorghum bicolor*), en surexprimant la L-aspartate oxydase codée par un acide nucléique choisi dans le groupe constitué par SEQ ID N°1, SEQ ID N°3, SEQ ID N°4, SEQ ID N°5, SEQ ID N°6, SEQ ID N°7, SEQ ID N°8, SEQ ID N°9, SEQ ID N°10, SEQ ID N°11, SEQ ID N°12, SEQ ID N°13, SEQ ID N°14, SEQ ID N°15, SEQ ID N°16 dans des cellules de plantes de sorgho.

La présente description divulgue la surexpression de la L-aspartate oxydase dans une dicotylédone, en particulier le coton (*Gossypium hirsutum*) en surexprimant la L-aspartate oxydase codée par un acide nucléique choisi dans le groupe constitué par SEQ ID N°1, SEQ ID N°3, SEQ ID N°4, SEQ ID N°5, SEQ ID N°6, SEQ ID N°7, SEQ ID N°8, SEQ ID N°9, SEQ ID N°10, SEQ ID N°11, SEQ ID N°12, SEQ ID N°13, SEQ ID N°14, SEQ ID N°15, SEQ ID N°16 dans des cellules de plantes de coton.

La présente description divulgue la surexpression de la L-aspartate oxydase dans une dicotylédone, en particulier la tomate (*Solanum lycopersicum*), en surexprimant la L-aspartate oxydase codée par un acide nucléique choisi dans le groupe constitué par SEQ ID N°1, SEQ ID N°3, SEQ ID N°4, SEQ ID N°5, SEQ ID N°6, SEQ ID N°7, SEQ ID N°8, SEQ ID N°9, SEQ ID N°10, SEQ ID N°11, SEQ ID N°12, SEQ ID N°13, SEQ ID N°14, SEQ ID N°15, SEQ ID N°16 dans des cellules de plantes de tomate.

La présente description divulgue la surexpression de la L-aspartate oxydase dans une dicotylédone, en particulier le colza (*Brassica napus*), en surexprimant la L-aspartate oxydase codée par un acide nucléique choisi dans le groupe constitué par SEQ ID N°1, SEQ ID N°3, SEQ ID N°4, SEQ ID N°5, SEQ ID N°6, SEQ ID N°7, SEQ ID N°8, SEQ ID N°9, SEQ ID N°10, SEQ ID N°11, SEQ ID N°12, SEQ ID N°13, SEQ ID N°14, SEQ ID N°15, SEQ ID N°16 dans des cellules de plantes de colza.

La présente description divulgue la surexpression de la L-aspartate oxydase dans une dicotylédone, en particulier le soja (*Glycine max*), en surexprimant la L-aspartate oxydase codée par un acide nucléique choisi dans le groupe constitué par SEQ ID N°1, SEQ ID N°3, SEQ ID N°4, SEQ ID N°5, SEQ ID N°6, SEQ ID N°7, SEQ ID N°8, SEQ ID N°9, SEQ ID N°10, SEQ ID N°11, SEQ ID N°12, SEQ ID N°13, SEQ ID N°14, SEQ ID N°15, SEQ ID N°16 dans des cellules de plantes de soja.

La présente description divulgue la surexpression de la L-aspartate oxydase dans une dicotylédone, en particulier le tournesol (*Helianthus annuus*), en surexprimant la L-aspartate oxydase codée par un acide nucléique choisi dans le groupe constitué par SEQ ID N°1, SEQ ID N°3, SEQ ID N°4, SEQ ID N°5, SEQ ID N°6, SEQ ID N°7, SEQ ID N°8, SEQ ID N°9, SEQ ID N°10, SEQ ID N°11, SEQ ID N°12, SEQ ID N°13, SEQ ID N°14, SEQ ID N°15, SEQ ID N°16 dans des cellules de plantes de tournesol.

La présente description divulgue la surexpression de la L-aspartate oxydase dans une dicotylédone, en particulier le haricot (*Phaseolus vulgaris*), en surexprimant la L-aspartate oxydase codée par un acide nucléique choisi dans le groupe constitué par SEQ ID N°1, SEQ ID N°3, SEQ ID N°4, SEQ ID N°5, SEQ ID N°6, SEQ ID N°7, SEQ ID N°8, SEQ ID N°9, SEQ ID N°10, SEQ ID N°11, SEQ ID N°12, SEQ ID N°13, SEQ ID N°14, SEQ ID N°15, SEQ ID N°16 dans des cellules de plantes de haricot.

La présente description divulgue la surexpression de la L-aspartate oxydase dans une dicotylédone, en particulier le peuplier (*Populus trichocarpa*), en surexprimant la L-aspartate oxydase codée par un acide nucléique choisi dans le groupe constitué par SEQ ID N°1, SEQ ID N°3, SEQ ID N°4, SEQ ID N°5, SEQ ID N°6, SEQ ID N°7, SEQ ID N°8, SEQ ID N°9, SEQ ID N°10, SEQ ID N°11, SEQ ID N°12, SEQ ID N°13, SEQ ID N°14, SEQ ID N°15, SEQ ID N°16 dans des cellules de plantes de peuplier.

L'homme du métier sait comment identifier des séquences d'acide nucléique codant pour la L-aspartate oxydase au sein de différentes espèces, par comparaison de SEQ ID N°1 avec les séquences d'autres espèces, avec un programme d'ordinateur tels que BLAST (www.ncbi.nlm.nih.gov) et DB rapide avec les paramètres par défaut. L'homme du métier pourra aussi utiliser les une des séquences d'acide nucléique choisie dans le groupe constitué par SEQ ID N°3, SEQ ID N°4, SEQ ID N°5, SEQ ID N°6, SEQ ID N°7, SEQ ID N°8, SEQ ID N°9, SEQ ID N°10, SEQ ID N°11, SEQ ID N°12, SEQ ID N°13, SEQ ID N°14, SEQ ID N°15, SEQ ID N°16 afin de réaliser des comparaisons de séquences afin d'identifier et trouver des séquences codant pour la L-aspartate oxydase convenables.

Ces algorithmes sont décrits dans «Les méthodes actuelles de séquençage et synthèse méthodes et applications», pages 127-149, 1988, en Alabama R. Liss, Inc.

Les séquences homologues sont de préférence définies comme suit :
i) des séquences d'ADN qui montrent une similarité ou identité d'au moins 55 %, de préférence au moins 70 %, de préférence au moins 80%, encore plus préférablement au moins 90 %, plus préférentiellement et encore au moins 95% avec la séquence SEQ ID N°1 ; ou avec une séquence choisie dans le groupe constitué par SEQ ID N°3, SEQ ID N°4, SEQ ID N°5, SEQ ID N°6, SEQ ID N°7, SEQ ID N°8, SEQ ID N°9, SEQ ID N°10, SEQ ID N°11, SEQ ID N°12, SEQ ID N°13, SEQ ID N°14, SEQ ID N°15, SEQ ID N°16
ii) des séquences qui s'hybrident avec la séquence de SEQ ID N°1, ou avec une séquence choisie dans le groupe constitué par SEQ ID N°3, SEQ ID N°4, SEQ ID N°5, SEQ ID N°6, SEQ ID N°7, SEQ ID N°8, SEQ ID N°9, SEQ ID N°10, SEQ ID N°11, SEQ ID N°12, SEQ ID N°13, SEQ ID N°14, SEQ ID N°15, SEQ ID N°16 ou avec sa séquence complémentaire, dans des conditions d'hybridation de stringence, par exemple de stringence faible, ou
iii) des séquences codant pour une enzyme L-aspartate oxydase comprenant la séquence d'acides aminés de la SEQ ID N°2, ou une séquence d'acides aminés homologue, par exemple, n'importe quelle séquence d'acides aminés avec une activité enzymatique L-aspartate oxydase et ayant au moins 60 %, de préférence au moins 70 %, de préférence au moins 80%, encore plus préférablement au moins 90%, plus préférentiellement encore au moins 95% d'identité de séquence avec la séquence de la SEQ ID N°2.

De manière préférentielle, une telle séquence nucléotidique homologue s'hybride spécifiquement à des séquences complémentaires de la séquence SEQ ID N°1 ou d'une séquence choisie dans le groupe constitué par SEQ ID N°3, SEQ ID N°4, SEQ ID N°5, SEQ ID N°6, SEQ ID N°7, SEQ ID N°8, SEQ ID N°9, SEQ ID N°10, SEQ ID N°11, SEQ ID N°12, SEQ ID N°13, SEQ ID N°14, SEQ ID N°15, SEQ ID N°16, dans des conditions rigoureuses. Les paramètres qui définissent les conditions de stringence dépendent de la température (Tm) à laquelle 50 % des brins appariés se séparent. Des conditions d'hybridation à faible stringence sont celles dans lesquelles l'hybridation est observée en utilisant une température d'hybridation de 5 à 10 °C en dessous de Tm, et les tampons d'hybridation sont des solutions de force ionique élevée, par exemple une solution 6 x SSC.

Les termes «similarité de séquence» ou «identité de séquence» ou encore «homologie de séquence» sont utilisés ici de manière interchangeable et signifient dans le contexte de deux ou plusieurs séquences d'acides nucléiques ou protéiques qui sont les mêmes ou ont un pourcentage spécifié de résidus d'acides aminés ou de nucléotides qui sont les mêmes, lorsque comparées et alignées pour une correspondance maximale, comme mesuré en utilisant l'un des algorithmes de comparaison de séquences suivants ou par inspection visuelle, si deux séquences devant être comparées entre elles ont des longueurs différentes, une identité de séquence concerne de préférence le pourcentage des résidus de nucléotides de la séquence la plus courte, qui sont identiques aux résidus nucléotidiques de la séquence plus longue. L'identité de séquence peut être déterminée de manière classique avec l'utilisation de programmes informatiques comme le programme Bestfit (Wisconsin Sequence Analysis Package, Version 8 pour Unix, Genetics Computer Group, University Research Park, 575 Sciences Drive Madison, WI 53711). Bestfit utilise l'algorithme d'homologie locale de Smith et Waterman (1981), afin de trouver le segment ayant l'identité de séquence la plus élevée entre les deux séquences. Quand on utilise Bestfit ou tout autre programme d'alignement de séquence pour déterminer si une séquence particulière présente, par exemple, 95% d'identité avec une séquence de la présente invention, de référence, les paramètres sont de préférence adaptés de telle manière que le pourcentage d'identité est calculé sur la longueur entière de la séquence de référence et que les écarts d'homologie allant jusqu'à 5 % du nombre total des nucléotides dans la séquence de référence sont permises.

Un acide nucléique est homologue à une séquence, telle que la séquence (séquence codante, CDS) représentée sur la SEQ ID N°1 par exemple, telle qu'utilisée ici, quand il comprend une séquence nucléotidique qui diffère de cette séquence, par exemple la SEQ ID N°1, par une mutation, insertion, délétion ou substitution d'une ou plusieurs bases, ou par la dégénérescence du code génétique, pour autant qu'elle code pour un polypeptide ayant l'activité de l'enzyme L-aspartate oxydase. Une protéine est homologue à la L-aspartate oxydase représentée dans la SEQ ID N°2, quand elle comprend une séquence d'acides aminés qui diffère de la séquence SEQ ID N°2, par mutation, insertion, délétion ou substitution d'un ou plusieurs des acides aminés, du moment qu'elle est un polypeptide ayant l'activité de l'enzyme L-aspartate oxydase.

La L-aspartate oxydase (EC 1.4.3.16) est une enzyme qui catalyse la réaction chimique :

L-aspartate + H₂O + O₂ → iminoaspartate + H₂O₂

Les trois substrats de cette enzyme sont L-aspartate, H₂O et O₂, alors que ses deux produits sont iminoaspartate et H₂O₂ En solution à pH8, l'iminoaspartate produit lors de la réaction enzymatique, encore appelé iminosuccinate, est instable, et produit du NH₄⁺ (demie-vie : 2,5 min).

La L-aspartate oxydase est une enzyme qui catalyse la première étape de la biosynthèse de novo de NAD⁺. L'oxygène peut être remplacé par le fumarate comme accepteur d'électrons, pour donner le succinate. La capacité de l'enzyme à utiliser à la fois l'oxygène et le fumarate comme cofacteur de ré-oxydation lui permet de fonctionner dans des conditions aérobies et anaérobies. L'enzyme est un membre de la famille des enzymes succinate déshydrogénase / fumarate-réductase.

L'expression «l'activité de l'enzyme L-aspartate oxydase» ou «l'activité enzymatique L-aspartate oxydase», telle qu'utilisée ici, se réfère en particulier à son activité oxydoréductase dans les plantes, qui peut être déterminée par incubation de la protéine avec L-Aspartate, fumarate et FAD pendant 30 min, suivi de la mesure spectrophotométrique à DO 635nm du NH₄⁺ libéré par décomposition de l'iminoaspartate produit lors de la réaction. Un protocole détaillé de mesure est décrit dans la section expérimentale plus après.

L'acide nucléique qui code pour la L-aspartate oxydase est généralement inséré, en simple ou multiples exemplaires, dans une construction nucléotidique, appelée cassette d'expression, dans laquelle il est lié de manière fonctionnelle à des éléments permettant son expression, plus particulièrement sa surexpression et éventuellement sa régulation.

Parmi ces éléments, on peut notamment citer les promoteurs de transcription, des activateurs et / ou terminateurs.

Dans un mode de réalisation particulier, la cellule végétale est transformée avec une cassette d'expression comprenant au moins une séquence nucleotidique codant la L-aspartate oxydase et un promoteur spécifique d'un tissu. L'expression dans les tissus contenant de la lignine ou en inflorescences peut être d'un intérêt particulier, ainsi que l'expression sélective ou préférentielle dans les fleurs ou les graines. Un promoteur spécifique de la racine peut également être utile. L'expression dans les tissus des racines peut être accompli en utilisant le gène de chitinase acide (Samac et al., 1990), ou les sous-domaines spécifiques profonds du promoteur CaMV35S qui ont été identifiés (Benfield et al., 1989).

Parmi les promoteurs de transcription qui peuvent être employés, on peut citer : un promoteur 35S, ou le promoteur 35S à double constitutive (pd35S) du virus de la mosaïque du chou-fleur (CaMV), comme décrit dans Kay et al, 1987 ; un promoteur PCRU de la cruciférine de radis qui dirige l'expression des séquences associées uniquement dans les semences de la plante transgénique ; les PGEA1 et PGEA6 promoteurs qui correspondent à la région 5' non codante des gènes de la protéine de stockage de graines (GEA1 et GEA6, respectivement) *d'Arabidopsis thaliana* qui dirigent une expression spécifique dans les graines ; le promoteur chimérique PSP (Ni et al., 1995) qui est une fusion d'une triple répétition d'un élément activateur de la transcription du promoteur du gène de l'octopine synthase dans *Agrobacterium tumefaciens* ; un promoteur d'actine de riz, suivi éventuellement par l'intron actine de riz (PAR- IAR), par exemple ; le promoteur contenu dans le plasmide pAct1-F4 (Mc Elroy et al, 1991) la HMGW promoteur de blé (de haut poids moléculaire gluténine) ; le promoteur du gène de la zéine du maïs (P-zéine) contenu dans le plasmide p63, qui dirige l'expression dans l'albumen des graines.

D'autres promoteurs exprimables dans une plante convenables conformément à la présente invention comprennent, mais ne sont pas limités à : des promoteurs provenant de la famille de l'ubiquitine (par exemple, le promoteur de l'ubiquitine du maïs du document EP 0 342 926), un promoteur actine du riz tels que le promoteur décrit par Mc Elroy et al., (déjà mentionné ci-dessus) ou le promoteur décrit dans US 5,641,876 ; un quelconque des promoteurs de la veine virus de la mosaïque du manioc (WO 97/48819), l'un quelconque de la série de promoteurs pPLEX de trèfle souterrain Stunt virus (WO 96/06932), ou un promoteur de l'alcool déshydrogénase, par exemple, pADH 1S (numéros d'accession GenBank X04049, X00581).

Parmi les terminateurs utilisables dans les constructions de l'invention, on peut notamment citer l'extrémité 3' du gène de la nopaline synthase *d'Agrobacterium tumefaciens.*

La cassette d'expression peut être insérée dans un vecteur nucléotidique, tel qu'un plasmide, qui peut en outre comprendre un gène marqueur, par exemple un gène permettant de sélectionner une plante transformée d'une plante qui ne contient pas l'ADN étranger transfecté. En tant que gène marqueur, celui-ci peut en particulier être constitué d'un gène qui confère une résistance à un antibiotique ou une résistance à un herbicide, ou une résistance à un acide aminé par exemple.

Le vecteur ainsi construit peut être utilisé pour transformer des cellules hôtes, selon des techniques connues de l'homme du métier.

On peut notamment citer des méthodes de transfert direct de gènes dans des cellules végétales, telles que la transformation par *Agrobacterium tumefaciens,* la micro-injection directe dans des embryoïdes de plante (Neuhaus et al., 1987), l'infiltration sous vide (Bechtold et al.,1993) ou l'électroporation (Chupeau et al., 1989), ou en variante, la précipitation directe à l'aide de PEG (Schocher et al., 1986) ou le bombardement de particules recouvertes de l'ADN plasmidique d'intérêt, en utilisant un pistolet (Fromm M. et al., 1990), par exemple.

Dans un mode de réalisation ici décrit , les cellules végétales sont transformées avec un vecteur tel que défini ci-dessus, transféré dans un hôte cellulaire susceptible d'infecter lesdites cellules végétales en permettant l'intégration, dans le génome de ces dernières, des séquences nucléotidiques d'intérêt initialement contenues dans le génome du vecteur susmentionné. Avantageusement, l'hôte cellulaire utilisé est une souche bactérienne, telle que *Agrobacterium tumefaciens,* ou *Agrobacterium rhizogenes* par exemple.

Pour transformer les monocotylédones telles que le riz (*Oryza sativa),* le procédé décrit par Ishida et al., (1996) peut être utilisé ou l'une quelconque des méthodes décrites dans Hiei et al., (1994), Hiei et al. (1997), dans le brevet US 5.641.664 ou 5.679.558, ou dans Christou et al., (1991). Selon un autre protocole, la transformation peut être réalisée selon la méthode décrite par Finer et al., (1992) en utilisant un canon à particules avec des particules de tungstène ou d'or, par exemple.

Les plantes ainsi obtenues surexpriment la L-aspartate oxydase.

De telles plantes ou parties d'une plante sont avantageusement obtenues par le procédé décrit ci-dessus, dans lequel une cellule végétale est transformée avec au moins un acide nucléique qui code pour l'enzyme L-aspartate oxydase, et mises en culture, grâce à quoi on obtient une plante qui surexprime la L-aspartate oxydase.

Comme exemples de plantes transgéniques, on peut citer blé, orge, riz, maïs, sorgho, tournesol, colza, soja, coton, pois, haricot, manioc, mangue, banane, pomme de terre, tomate, poivron, melon, courgette, pastèque, laitue, choux, aubergine, peuplier.

L'augmentation de la croissance des plantes, en particulier des racines, favorise la vigueur des plantes et leur capacité à récupérer les substrats nutritifs et l'eau dans le sol ou le milieu de culture.

La vitesse de croissance et l'augmentation de la vitesse de croissance des plantes, en particulier des inflorescences et des fruits sont avantageuses pour la production de grains, de fourrage, de fleurs ou de fruits, en particulier de légumes-fruits pour les cultures potagères.

Les plantes transgéniques selon l'invention englobent à la fois les plantes de la première génération ainsi que leurs descendants contenant la cassette d'expression permettant la surexpression de la L-aspartate oxydase selon l'invention (variétés lignées ou variétés hybrides, en particulier).
Les parties de la plante comprennent tout tissu ou organe, telles que des racines, des fleurs, des tiges, du tronc, des feuilles, des fruits, des organes de réserve ou des graines.

La présente description divulgue les graines qui présentent une expression accrue de L-aspartate oxydase, obtenue par une surexpression spécifique d'une séquence de codage de L-aspartate oxydase dans la graine.

Dans un mode de réalisation ici décrit, la cassette d'expression de l'invention est utilisée pour surexprimer la L-aspartate oxydase dans une plante ou une cellule végétale. Cette utilisation conduit à une augmentation de la biomasse, la croissance de la plante, la taille, le poids, le rendement et / ou la vitesse de croissance.

Dans un autre mode de réalisation ici décrit, la cassette d'expression selon l'invention est utilisée pour augmenter au moins un caractère phénotypique choisi parmi la biomasse, la vitesse de germination, le rendement, en particulier le rendement en grains, la résistance au stress abiotique, la résistance au stress biotique, la vitesse de germination, la croissance, dans une cellule végétale, une plante ou une partie de plante.

Comme évoqué plus avant, l'acide nucléique codant pour une surexpression de la L-aspartate oxydase au sein des plantes selon l'invention peut appartenir à la même espèce et ce dans la mesure où il peut être exprimé dans les plantes en des quantités supérieures à la quantité classiquement produite par une plante qui ne contenait pas la séquence nucléotidique ou l'élément génétique introduit. Ainsi, toute séquence nucléotidique codant pour la L-aspartate oxydase peut être utilisée, par exemple, une séquence de type sauvage pour le gène de la L-aspartate oxydase, une séquence mutée d'une séquence sauvage qui code pour la L-aspartate oxydase, ou encore une séquence mutée du promoteur du gène d'une L-aspartate oxydase sauvage ou mutée qui induit une augmentation de la quantité de L-aspartate oxydase, ou de la stabilité de l'ARN messager de la L-aspartate oxydase, ou une expression d'une L-aspartate oxydase ayant une activité enzymatique supérieure, ce par rapport à la quantité et/ou l'activité de L-aspartate oxydase qui étaient produites ou exprimées au sein de la plante hôte avant de recevoir la séquence introduite.

Comme indiqué plus avant, la surexpression de la L-aspartate oxydase dans les cellules de la plante peut être obtenue par divers moyens à la disposition de l'homme du métier, que ce soit par transgénèse, par transformation, par introgression, par sélection, par sélection assistée par marqueurs, par mutagénèse aléatoire ou dirigée suivie ou non de sélection, par exemple.

La présente description divulgue une surexpression de la L-aspartate oxydase réalisée par introgression d'un élément génétique codant pour une surexpression de la L-aspartate oxydase.

L'expression « élément génétique » ou « matériel génétique » utilisée ici se réfère à tout gène, groupe de gènes, QTL, locus, allèle, fragment chromosomique, séquence nucléotidique, séquence nucléique qui est capable de contribuer à l'augmentation d'au moins un caractère phénotypique de la plante, choisi parmi une biomasse accrue, une vitesse de germination accrue, un rendement accru, en particulier un rendement en grains accru, une résistance au stress abiotique accrue, une résistance au stress biotique accrue, une vitesse de germination accrue, une croissance accélérée, en influant sur l'expression de la L-aspartate oxydase au niveau de l'ADN lui-même, tant au niveau de la traduction, de la transcription et/ou de l'activation d'un produit de polypeptide final, c'est à dire, pour réguler le métabolisme de la plante conduisant à l'expression phénotypique du génotype.

Dans le contexte de la présente description, les termes «introgression», «introgressé» et «introgresser» désignent le processus par lequel un ou des éléments génétiques tels qu'un gène ou des gènes, un ou des QTL, un ou des allèles, un ou des fragments chromosomiques, ou une ou plusieurs séquences nucléiques présents dans le génome d'une espèce, variété ou un cultivar sont déplacés et transférés de manière stable dans le génome d'une autre espèce, variété ou cultivar, par croisement sexuel. Le transfert peut être naturel ou artificiel. Le processus peut éventuellement être complété par rétrocroisement avec un parent récurrent, dans ce cas, l'introgression se réfère à l'introduction d'un ou plusieurs éléments génétiques, tels qu'un ou plusieurs gènes, un ou plusieurs allèles, un ou plusieurs QTL, un ou plusieurs fragments chromosomiques ou une ou plusieurs séquences nucléiques d'une espèce dans le pool génétique de l'autre par rétrocroisement répété d'un hybride interspécifique avec l'un de ses parents. Une introgression peut également être décrite comme l'intégration stable d'un matériel génétique hétérologue dans le génome d'une plante receveuse par croisement sexuel entre plantes d'espèce identique ou proche, c'est-à-dire sexuellement compatibles. La notion de sexuellement compatible s'entend en ce que la fertilisation d'une fleur d'une plante par le pollen d'une autre plante donne lieu à la fécondation de l'ovule et à la production d'un fruit contenant une ou plusieurs graines capable de germer et de donner une nouvelle plante. La notion de sexuellement compatible s'entend aussi des cas ou la viabilité de l'embryon formé est assurée par une ou des techniques de sauvetage d'embryon. L'homme du métier dispose de diverses méthodes de sauvetage d'embryon qu'il peut utiliser en fonction des espèces mise en jeu.

Il est ainsi décrit une méthode pour augmenter au moins un caractère phénotypique choisi parmi la biomasse, la vitesse de germination, le rendement, en particulier le rendement en grains, la résistance au stress abiotique, la résistance au stress biotique, la vitesse de germination, la croissance, chez une plante comprenant la fourniture d'une population de plantes, y compris la fourniture d'une population de plantes issues de croisements, et la sélection des individus de la population qui présentent une expression de la L-aspartate oxydase la plus élevée possible.

La population de plante peut être une population de plantes mutantes générée par mutagénèse chimique ou par tout autre moyen capable d'induire une ou plusieurs mutations au sein du génome des plantes ainsi traitées. Le traitement de mutagénèse résulte en l'introduction volontaire de mutations par l'action d'agents mutagènes chimiques ou physiques dans une séquence ADN, agents qui peuvent être un traitement chimique comme par exemple un traitement à l'éthyl méthanesulfonate (EMS).

De manière avantageuse, une population TILLING peut être utilisée pour la sélection d'individus qui présentent une expression de la L-aspartate oxydase la plus élevée possible.

Typiquement la technologie TILLING repose sur la mutagénèse de graines suivie d'un phénotypage et d'un génotypage afin d'identifier les mutations et dont le ou les allèles associés à un phénotype donné, préférentiellement un phénotype avantageux. La génération de population TILLING peut être réalisée comme suit. Des graines M0 sont mutagénéisées par un traitement à l'ethylmethanosulfonate (EMS). Les plantes M1 issues des graines M0 sont autofécondées, l'ADN est extrait des familles M2 pour un screening haut débit des mutations et les graines M3 sont récoltées et conservées. L'ADN des familles M2 est poolé 8 fois et amplifié pour un gène cible. Les produits d'amplification sont incubés avec une endonucléase qui coupe préférentiellement les mésappariements dans les hétéroduplex entre sauvage et mutant. Les produits de digestion sont soumis à une électrophorèse en gel de séquence. La technologie LI-COR permet un marquage fluorescent double brin (IRDye 700 et 800) qui permet une confirmation visuelle rapide car les mutations sont détectées sur les deux brins complémentaires et ainsi facilement distinguées des artéfacts. Après détection d'une mutation dans un pool, l'ADN des familles individuelles est rapidement criblé par déconvolution du pool afin d'identifier la famille portant la mutation.

Dans un mode de réalisation ici décrit,, l'introgression d'un élément génétique codant pour une surexpression de la L-aspartate oxydase est obtenue par fusion de protoplastes.

Ainsi dans un tel mode de réalisation décrit, pour augmenter au moins un caractère phénotypique choisi parmi la biomasse, la vitesse de germination, le rendement, en particulier le rendement en grains, la résistance au stress abiotique, la résistance au stress biotique, la vitesse de germination, la croissance, la fusion de protoplastes peut être utilisée pour le transfert d'au moins un élément génétique à partir d'une plante donneuse vers une plante receveuse. La fusion de protoplastes est une union induite ou spontanée, comme une hybridation somatique, entre deux ou plusieurs des protoplastes (les parois cellulaires sont éliminées par traitement enzymatique) afin de produire une cellule unique bi- ou multi- nucléée. La cellule fusionnée, qui peut aussi être obtenue avec les espèces végétales qui ne peuvent être croisées sexuellement dans la nature, est cultivée dans une plante hybride présentant la combinaison de caractéristiques souhaitables. Plus précisément, un premier protoplaste peut être obtenu à partir d'un plante selon l'invention et qui présente au moins un caractère phénotypique choisi parmi la biomasse, la vitesse de germination, le rendement, en particulier le rendement en grains, la résistance au stress abiotique, la résistance au stress biotique, la vitesse de germination, la croissance et une surexpression de la L-aspartate oxydase. Un deuxième protoplaste peut être obtenu à partir d'une plante qui comprend des caractéristiques de valeur commerciale. Les protoplastes sont ensuite fusionnés au moyen de procédures de fusion de protoplastes traditionnelles, qui sont connues dans la technique.

Alternativement, le sauvetage d'embryon peut être utilisé dans le transfert d'un élément génétique, en particulier un acide nucléique, permettant la surexpression de la L-aspartate oxydase d'une plante donneuse vers une plante receveuse. Le sauvetage d'embryons peut être utilisé en tant que procédure pour isoler des embryons à partir de croisements dans lequel les plantes ne parviennent pas à produire des graines viables. Dans ce processus, l'ovule fécondé ou immature d'une plante est un tissu de culture pour créer de nouvelles plantes.

La présente description divulgue une méthode dans laquelle l'introgression d'un élément génétique codant pour une surexpression de la L-aspartate oxydase est obtenue par sauvetage d'embryon.

La présente description divulgue une méthode pour la production de plantes présentant au moins un caractère phénotypique amélioré choisi parmi la biomasse, la vitesse de germination, le rendement, en particulier le rendement en grains, la résistance au stress abiotique, la résistance au stress biotique, la vitesse de germination, la croissance, comprenant la détection de la présence d'un élément génétique, en particulier une séquence d'acide nucléique, lié à la surexpression de la L-aspartate oxydase dans une plante donneuse et le transfert de l'élément génétique, en particulier une séquence d'acide nucléique, lié à la surexpression de la L-aspartate oxydase ainsi détecté, depuis la plante donneuse vers une plante receveuse. Le transfert de la séquence d'acide nucléique peut être réalisé par un quelconque des procédés précédemment décrits ici.

Le transfert peut être réalisé par une technique choisie parmi transgénèse, introgression, fusion de protoplastes, restauration d'embryon. Un exemple de réalisation d'un tel procédé comprend le transfert par introgression de l'élément génétique, en particulier la séquence d'acide nucléique, lié à la surexpression de la L-aspartate oxydase à partir d'une plante donneuse vers une plante receveuse par croisement sexuel des plantes. Ce transfert peut donc avantageusement être réalisé en utilisant des techniques de croisement et de sélection traditionnelles.

La présente description divulgue la détection de la présence d'un élément génétique lié à la surexpression de la L-aspartate oxydase et est réalisée à l'aide d'au moins un marqueur moléculaire.

La présente description divulgue la détection de la présence de l'élément génétique lié à la surexpression de la L-aspartate oxydase qui est réalisée par la mesure de l'activité enzymatique de la L-aspartate oxydase dans la plante donneuse.

Selon certains mode de réalisation, l'élément génétique responsable de la surexpression de la L-aspartate oxydase peut être introgressé dans les variétés commerciales de plantes d'intérêt agronomique à l'aide de la sélection assistée par marqueurs (SAM) qui implique l'utilisation d'un ou plusieurs des marqueurs moléculaires pour l'identification et la sélection des plantes de la descendance qui contiennent l'élément génétique, gène ou pluralité de gènes, séquences d'acide nucléique codant pour la caractéristique souhaitée de surexpression de la L-aspartate oxydase.

Dans le contexte de la présente description, une telle identification et une telle sélection sont basées sur la sélection de gènes, d'éléments génétiques ou de séquences d'acides nucléiques ou des marqueurs qui y sont associés.

Les plantes obtenues selon ces modes de réalisation peuvent avantageusement tirer la majorité de leurs traits de la plante receveuse, et tirer au moins un caractère phénotypique choisi parmi la biomasse accrue, la vitesse de germination accrue, le rendement accru, en particulier le rendement en grains accru, la résistance au stress abiotique accrue, la résistance au stress biotique accrue, la vitesse de germination accrue, la croissance accrue de la plante donneuse, grâce à la surexpression de la L-aspartate oxydase.

Comme discuté ci-dessus, les techniques de croisement traditionnelles peuvent être utilisées pour l'introgression de séquence d'acide nucléique responsable de la surexpression de la L-aspartate oxydase liée à au moins un caractère phénotypique choisi parmi la biomasse accrue, la vitesse de germination accrue, le rendement accru, en particulier le rendement en grains accru, la résistance au stress abiotique accrue, la résistance au stress biotique accrue, la vitesse de germination accrue, la croissance accrue au sein d'une plante de receveuse .

Dans certains modes de réalisation, une plante donneuse qui présente au moins un caractère phénotypique choisi parmi la biomasse accrue, la vitesse de germination accrue, le rendement accru, en particulier le rendement en grains accru, la résistance au stress abiotique accrue, la résistance au stress biotique accrue, la vitesse de germination accrue, la croissance accrue et comprenant une séquence d'acide nucléique responsable d'une surexpression de la L-aspartate oxydase, est croisée avec une plante receveuse qui, dans certains modes de réalisation peut présenter des caractéristiques commercialement souhaitables .

La population de plantes qui en résulte (représentant les hybrides F1) est alors autofécondée produisant des semences F2. Les plantes F2 issues de graines F2 sont ensuite criblées en vue de déterminer les plantes présentant une surexpression de la L-aspartate oxydase, associée à au moins un caractère phénotypique choisi parmi la biomasse accrue, la vitesse de germination accrue, le rendement accru, en particulier le rendement en grains accru, la résistance au stress abiotique accrue, la résistance au stress biotique accrue, la vitesse de germination accrue, la croissance accrue ; ceci par des méthodes connues de l'homme du métier.

La mesure de l'expression ou de la surexpression de la L-aspartate oxydase peut être réalisée par différents moyens à la disposition de l'homme du métier tels que RNA-Seq, Northern blot, PCR quantitative et semi-quantitative, Western blot, Elisa ou mesure d'activité enzymatique, par exemple.

Les lignées de plantes présentant une surexpression de la L-aspartate oxydase, associée à au moins un caractère phénotypique choisi parmi la biomasse accrue, la vitesse de germination accrue, le rendement accru, en particulier le rendement en grains accru, la résistance au stress abiotique accrue, la résistance au stress biotique accrue, la vitesse de germination accrue, la croissance accrue, peuvent être développées en utilisant les techniques de sélection récurrente et rétrocroisement, autofécondation, et/ou dihaploïdes, ou toute autre technique utilisée pour faire des lignées parentales. Dans un procédé de sélection récurrente et rétrocroisement, l'augmentation de l'expression de la L-aspartate oxydase, associée à au moins un caractère phénotypique choisi parmi la biomasse, la vitesse de germination, le rendement, en particulier le rendement en grains, la résistance au stress abiotique, la résistance au stress biotique, la vitesse de germination, la croissance, peut être introgressée dans une plante receveuse cible (le parent récurrent) en croisant le parent récurrent avec une première plante donneuse, qui diffère du parent récurrent et qui est appelée ici le «parent non récurrent». Le parent récurrent est une plante qui ne présente pas une surexpression de la L-aspartate oxydase, associée à au moins un caractère phénotypique choisi parmi la biomasse accrue, la vitesse de germination accrue, le rendement accru, en particulier le rendement en grains accru, la résistance au stress abiotique accrue, la résistance au stress biotique accrue, la vitesse de germination accrue, la croissance accrue, mais peut posséder des caractéristiques commerciales souhaitables.

Le parent non récurrent peut être n'importe quelle variété végétale ou lignée pure qui est sexuellement compatible avec le parent récurrent.

Les plantes de la descendance d'un croisement entre le parent récurrent et le parent non récurrent sont rétrocroisées au parent récurrent. La population de plantes qui en résulte est ensuite criblée pour une surexpression de la L-aspartate oxydase, associée à au moins un caractère phénotypique choisi parmi la biomasse accrue, la vitesse de germination accrue, le rendement accru, en particulier le rendement en grains accru, la résistance au stress abiotique accrue, la résistance au stress biotique accrue, la vitesse de germination accrue, la croissance accrue.

La sélection assistée par marqueurs (SAM) peut être mise en œuvre en utilisant des sondes d'hybridation ou des polynucléotides, afin d'identifier les plantes qui comprennent une séquence d'acide nucléique ou tout élément génétique entraînant une surexpression de la L-aspartate oxydase, associée à au moins un caractère phénotypique choisi parmi la biomasse accrue, la vitesse de germination accrue, le rendement accru, en particulier le rendement en grains accru, la résistance au stress abiotique accrue, la résistance au stress biotique accrue, la vitesse de germination accrue, la croissance accrue.

À la suite de criblage, les plantes hybrides F1 qui présentent une surexpression de la L-aspartate oxydase, associée à au moins un caractère phénotypique choisi parmi la biomasse accrue, la vitesse de germination accrue, le rendement accru, en particulier le rendement en grains accru, la résistance au stress abiotique accrue, la résistance au stress biotique accrue, la vitesse de germination accrue, la croissance accrue, sont ensuite sélectionnées et rétrocroisées avec le parent récurrent pour un certain nombre de générations afin de permettre à la plante de devenir de plus en plus consanguine. Ce processus peut être effectué pour deux, trois, quatre, cinq, six, sept, huit, ou plus générations .

La présente description divulgue un procédé d'obtention de plante présentant au moins un caractère phénotypique choisi parmi la biomasse accrue, la vitesse de germination accrue, le rendement accru, en particulier le rendement en grains accru, la résistance au stress abiotique accrue, la résistance au stress biotique accrue, la vitesse de germination accrue, la croissance accrue qui peut comprendre :
(a) fournir une plante présentant un niveau d'expression donné de la L-aspartate oxydase ;
(b) fournir une seconde plante présentant un niveau d'expression de la L-aspartate oxydase supérieur à celui de la plante fournie en (a) ;
(c) effectuer un croisement de la plante fournie en (a) avec la plante fournie en (b), pour produire des plantes de la descendance F1 ;
(d) sélectionner des plantes de la descendance F1 qui présentent une surexpression de la L-aspartate oxydase par rapport à la plante fournie en (a) ;
(e) croiser les plantes sélectionnées en (d) avec la plante fournie en (a) pour produire des plantes de descendance de rétrocroisement ;
(f) sélectionner des plantes de la descendance de rétrocroisement qui présentent une surexpression de la L-aspartate oxydase par rapport aux plantes sélectionnées en (d);
(g) répéter les étapes (e) et (f) deux ou plusieurs fois de suite;
(h) éventuellement autoféconder les plantes issues de rétrocroisement afin d'identifier les plantes homozygotes et
(i) effectuer un croisement d'au moins une plante de la descendance de rétrocroisement ou des plantes auto-fécondées avec une autre plante fournie en (a) pour produire une plante présentant au moins un caractère phénotypique choisi parmi la biomasse accrue, la vitesse de germination accrue, le rendement accru, en particulier le rendement en grains accru, la résistance au stress abiotique accrue, la résistance au stress biotique accrue, la vitesse de germination accrue, la croissance accrue lorsque cultivée dans les mêmes conditions environnementales.

Comme indiqué, la dernière génération de rétrocroisement peut être auto-fécondée afin de fournir des individus homozygotes présentant une surexpression de la L-aspartate oxydase et au moins un caractère phénotypique choisi parmi la biomasse accrue, la vitesse de germination accrue, le rendement accru, en particulier le rendement en grains accru, la résistance au stress abiotique, la résistance au stress biotique accrue, la vitesse de germination accrue, la croissance accrue.

L'étape de sélection comprend la sélection d'individus qui contiennent chacune un allèle du gène qui code pour une surexpression de la L-aspartate oxydase.

La présente description divulgue une méthode de sélection de plante, caractérisé en ce qu'elle comprend la recherche d'un allèle du gène de l'enzyme L-aspartate oxydase possédant une mutation résultant en une amélioration d'au moins un caractère phénotypique choisi parmi biomasse, la vitesse de germination, le rendement, en particulier le rendement en grains, la résistance au stress abiotique, la résistance au stress biotique, la vitesse de germination, la croissance.

L'étape de sélection comprend la sélection à l'aide d'un marqueur moléculaire pour l'allèle du gène de la L-aspartate oxydase.

L'étape de sélection comprend la sélection en mesurant l'activité de l'enzyme L-aspartate oxydase dans les jeunes plantes et en sélectionnant celles présentant une activité élevée de l'enzyme.

La présente description divulgue un procédé de sélection de plante présentant une surexpression de la L-aspartate oxydase comprenant les étapes suivantes :
(a) fournir une population de plantes ;
(b) mesurer l'activité de la L-aspartate oxydase de chaque individu de la population de plantes fournie ;
(c) sélectionner la ou les plantes présentant l'activité L-aspartate oxydase la plus élevée.

La population de plante peut être une population de TILLING.

Alternativement, la population de plantes est une population issue de croisements intraspécifiques ou interspécifiques.

Aussi la population de plantes peut être une population de variétés commerciales.

Dans le contexte de la présente invention, l'expression «surexpression» en référence à la L-aspartate oxydase se réfère au fait que la séquence nucléique d'ADN encodant la protéine est transcrite en ARN en quantité accrue et/ou que les ARN de la protéine sont traduits en protéine en quantité accrue et/ou que la quantité ou l'activité spécifique de la protéine traduite est accrue. La mesure de cette « surexpression » peut être évaluée tant au niveau de l'ARN qu'au niveau des protéines par différents moyens à la disposition de l'homme du métier tels que RNA-Seq, Northern blot, PCR quantitative et semi-quantitative, Western blot, Elisa ou mesure d'activité enzymatique, par exemple. Au final, la notion d'expression ou de surexpression fait référence à l'activité enzymatique de la L-aspartate oxydase au sein de la plante et ce qu'elle soit due à une plus grande synthèse de la protéine et/ou à activité spécifique plus importante.

Dans le contexte de la présente description, les expressions «croisement sexuel» et «reproduction sexuée» se réfèrent à la fusion des gamètes pour produire une descendance (par exemple par fécondation, de manière à produire des semences par pollinisation chez les plantes). Un «croisement sexuel» ou «fertilisation croisée» est dans certains modes de réalisation, la fécondation d'un individu par un autre (par exemple, la pollinisation croisée des plantes). Le terme «autofécondation» fait référence à certains modes de réalisation de la production de semences par autofécondation ou auto-pollinisation, c'est-à-dire que pollen et ovules sont de la même plante.

Par l'expression «caractère», on entend une caractéristique ou un phénotype, par exemple le rendement, la biomasse ou la vitesse de germination. Un caractère peut être hérité de manière dominante ou récessive, ou peut être monogénique ou polygénique.

Par l'expression «plante donneuse», on entend une plante qui fournit au moins un élément génétique lié à une surexpression de la L-aspartate oxydase.

Par l'expression «plante receveuse», on entend une plante qui reçoit au moins un élément génétique lié à une surexpression de la L-aspartate oxydase.

Les expressions «marqueur génétique», «marqueur d'ADN» ou «marqueur moléculaire» sont interchangeables et font référence à une caractéristique du génome d'un individu (par exemple un nucléotide ou une séquence de d'acide nucléique qui est présent dans le génome d'un individu), qui est liée à un ou plusieurs loci d'intérêt. Les marqueurs génétiques comprennent, par exemple, des polymorphismes nucléotidiques simples (SNP), des indels (c-à-d insertions/suppressions), des répétitions de séquences simples (SSR), des polymorphismes de restriction de longueur des fragments (RFLP), ou des longueurs des fragments amplifiés (AFLP) par exemple. Les marqueurs génétiques peuvent, par exemple, être utilisés pour localiser des loci génétiques contenant des allèles qui contribuent à la variabilité des caractéristiques phénotypiques. L'expression «marqueur génétique» peut également faire référence à une séquence de polynucléotide complémentaire d'une séquence génomique, telle qu'une séquence d'un acide nucléique utilisé comme sonde. Un marqueur génétique ou moléculaire peut être physiquement situé dans une position sur un chromosome qui est distale ou proximale par rapport à un ou des loci génétiques avec lequel ou lesquels il est lié (c'est à dire est intragénique ou extragénique, respectivement). Dans certains modes de réalisation de la présente invention, les un ou plusieurs marqueurs génétiques comprennent entre un et dix marqueurs, et dans certains modes de réalisation, les un ou plusieurs marqueurs génétiques comprennent plus d'une dizaine de marqueurs génétiques.

Le terme «génotype» fait référence à la constitution génétique d'une cellule ou un organisme. Comme cela est connu dans la technique, un génotype peut se rapporter à un seul locus ou à de multiples loci. Dans certains modes de réalisation, le génotype d'un individu se rapporte à un ou plusieurs gènes qui sont liés par le fait que l'un ou plusieurs des gènes sont impliqués dans l'expression d'un phénotype d'intérêt (par exemple un caractère tel que défini ici). Ainsi, dans certains modes de réalisation, un génotype comporte un ou plusieurs allèles présents chez un individu à un ou plusieurs loci pour un caractère.

Le terme «gène» se réfère à une unité héréditaire comprenant une séquence d'ADN qui occupe un emplacement spécifique sur un chromosome et qui contient l'instruction génétique pour une caractéristique ou un trait particulier dans un organisme.

Les termes «acide nucléique» ou «oligonucléotide» ou «polynucléotide» ou «séquence nucléique» ou des équivalents grammaticaux de ceux-ci, signifient au moins deux nucléotides liés de manière covalente ensemble. Les oligonucléotides sont typiquement d'environ 7, 8, 9, 10, 12, 15, 25 18, 20, 30, 40, 50 ou jusqu'à environ 100 nucléotides de long. Les acides nucléiques, séquences nucléiques et les polynucléotides sont des polymères de n'importe quelle longueur, y compris les plus grandes longueurs, par exemple, 200, 300, 500, 1000, 2000, 3000, 5000, 7000, 10000, etc. Un acide nucléique de la présente invention contiendra généralement des liaisons phosphodiester, bien que dans certains cas, des analogues d'acide nucléique sont inclus qui peuvent avoir des squelettes alternatifs comprenant, par exemple, phosphoramidate, phosphorothioate, phosphorodithioate, ou les liaisons O-methylphosphoroamidite (voir Eckstein, 1991), et des squelettes peptidiques et les liens d'acides nucléiques. Des mélanges d'acides nucléiques naturels et d'analogues peuvent être utilisés.

Dans le cadre de la présente invention, l'expression «phénotype» ou «caractère phénotypique» fait référence à l'apparence ou toute autre caractéristique détectable d'un individu, résultant de l'interaction de son génome, protéome et/ou métabolome avec l'environnement.

Une «plante» est une plante à n'importe quel stade de développement, en particulier une plante à graines.

Une «cellule de plante» est une unité structurale et physiologique d'une plante, comprenant un protoplaste et une paroi cellulaire. La cellule végétale peut être sous forme d'une cellule unique isolée ou une cellule cultivée, ou comme une partie d'unité organisée supérieure telle que, par exemple, un tissu végétal, un organe végétal ou une plante entière. Une cellule de plante peut être capable de régénérer une plante ou pas être capable de régénérer une plante.

L'expression «matériel végétal» se réfère à feuilles, tiges, racines, fleurs ou parties de fleurs, les fruits, le pollen, les ovules, les zygotes, graines, boutures, cellulaires ou cultures de tissus, ou de toute autre partie ou de produits d'une usine.

Telle qu'utilisée ici, l'expression «partie de plante» désigne une partie d'une plante, comprenant des cellules simples et des tissus cellulaires telles que les cellules de plantes qui sont intactes dans des plantes, des amas de cellules, et des cultures de tissu à partir duquel les plantes peuvent être régénérées ou pas. Des exemples de parties de plantes comprennent, mais ne sont pas limités à, des cellules individuelles et des tissus de pollen, les ovules, les feuilles, les embryons, les racines, bouts de racine, anthères, les fleurs, les fruits, les tiges, les pousses et les graines, ainsi que des greffons, porte-greffes, protoplastes, calii, et analogues.

Tel qu'il est utilisé ici, le terme «population» désigne un ensemble génétiquement hétérogène de plantes partageant une dérivation génétique commune.

La « biomasse » signifie l'ensemble de la matière organique produite par une plante. Cette biomasse peut être mesurée en poids frais, en poids sec par plante ou par m² par exemple. Alternativement, la biomasse peut être évaluée par la taille de la plante ou le nombre de feuilles par exemple.

L'expression « vitesse de germination » correspond au temps moyen entre l'imbibition de la graine et l'émergence de la radicule de l'enveloppe de la graine.

L'expression « rendement » s'entend comme la quantité de matière végétale commerciale produite par la plante cultivée par unité de surface, et ce pour une densité de plantation donnée le cas échéant.

L'expression «rendement en grains» représente la quantité de grains, en poids ou en nombre, produite par la plante cultivée par unité de surface ou par plante, et ce pour une densité de plantation donnée le cas échéant.

L'expression «résistance au stress biotique» s'entend comme la capacité de la plante à faire face et à lutter contre un stress qui survient à la suite de dommages causés à ladite plante par d'autres organismes vivants, comme les bactéries, les virus, les champignons, les parasites, les insectes utiles et nuisibles, les mauvaises herbes et les autres plantes cultivées ou indigènes. Les dommages causés par ces divers agents vivants peuvent apparaître très similaires et affectent la croissance et le rendement des plantes cultivées.

L'expression «résistance au stress abiotique» s'entend comme la capacité de la plante à faire face et à lutter contre un stress induit par des facteurs non-vivants dans un environnement spécifique. Les facteurs de stress abiotiques sont d'origine naturelle, souvent intangibles, des facteurs tels que la lumière du soleil intense, ou le déficit de lumière, l'excès ou le déficit d'eau, le froid ou l'excès de chaleur, la salinité ou encore le vent par exemple, peuvent causer des dommages aux plantes de la zone affectée. Le stress abiotique est essentiellement inévitable et particulièrement contraignant pour les plantes. Le stress abiotique est le facteur le plus nocif sur la croissance et la productivité des cultures dans le monde entier.

L'expression « croissance » fait référence à la variation de biomasse de la plante considérée, entre l'émergence et la récolte par unité de temps dans des conditions données d'ensoleillement, d'irrigation, et d'intrants.

### FIGURES

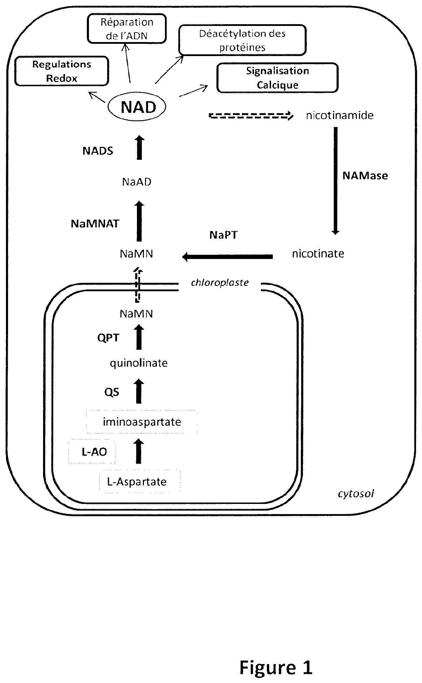

La figure 1 est une description de la voie de biosynthèse de NAD dans les plantes, et son utilisation pour le métabolisme énergétique et la signalisation liée au stress. (l'activité L-aspartate oxydase (AO) est mise en évidence dans la figure).

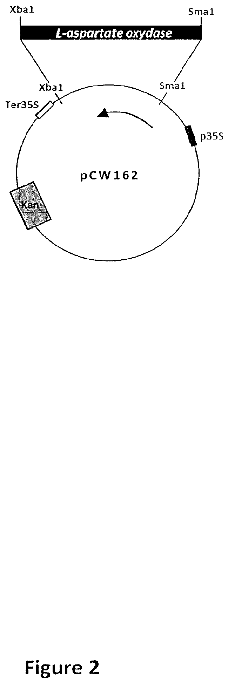

La figure 2 est une représentation schématique de la construction d'un vecteur de transformation de plante pCW162, y compris la cassette de surexpression de la L-aspartate oxydase (AO).
La figure 3 est un graphique représentant le niveau d'expression (A) et l'activité (B) de la L-aspartate oxydase dans les feuilles de plantes contrôle (ctrl), de plantes surexprimant la L-aspartate oxydase (35S::AO1 et 35S::AO2) et de plantes mutant-négatif de la L-aspartate oxydase (mAO), âgées de 6 semaines.
La figure 4 est un graphique représentatif des métabolites liés à l'énergie (ATP (B) et nucléotides à pyridine (A)) dans les feuilles de plantes contrôle (ctrl), de plantes surexprimant la L-aspartate oxydase (35S::AO1 et 35S::AO2) et de plantes mutant-négatif de la L-aspartate oxydase (mAO), âgées de 6 semaines.
La figure 5 représente les capacités photosynthétiques de feuilles de plantes contrôle ( 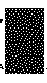 ) et de plantes surexprimant (▲) la L-aspartate-oxydase, âgées de 6 semaines.
La figure 6 est une photographie de plantes *d'Arabidopsis thaliana* contrôle et de de plantes surexprimant la L-aspartate oxydase (35S::AO1 et 35S::AO2), âgées de 6 semaines.
La figure 7 est un graphique représentant la biomasse (B) et la taille (A) des plantes contrôle (ctrl), de plantes surexprimant la L-aspartate oxydase (35S::AO1 et 35S::AO2) et de plantes mutant-négatif de la L-aspartate oxydase (mAO).
La figure 8 est un graphique représentant la biomasse en graines récoltées à partir de plantes contrôle (ctrl), de plantes surexprimant la L-aspartate oxydase (35S::AO1 et 35S::AO2) et de plantes mutant-négatif de la L-aspartate oxydase (mAO).
La figure 9 est un graphique représentant la corrélation entre l'activité de la L-aspartate oxydase (A), les niveaux de NAD (B) et la de biomasse exprimée en diamètre de rosette.
La figure 10 est une photographie de plantes contrôle (ctrl) et de plantes surexprimant la L-aspartate oxydase (35S::AO) dans des conditions de stress abiotique correspondant à une forte chaleur combinée à une forte luminosité.
La figure 11 est une courbe de germination de plantes contrôle (◆) et de plantes surexprimant la L-aspartate oxydase (35S::AO1 ( 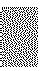 et X) et 35S::AO2 (▲)) dans des conditions de milieu riche en azote (nitrate) (A) et de conditions de milieu déficient en azote (nitrate) (B).
La figure 12 est un graphique représentant des conditions de stress biotique correspondant à la prolifération de pucerons *Myzus persicae* sur des plantes contrôle (ctrl) et des plantes surexprimant la L-aspartate oxydase (35S::AO1 et 35S::AO2).

### EXEMPLES

### Matériel et Méthodes

### Génération de plantes transgéniques

L'ADNc (séquence codante, CDS) *d'Arabidopsis thaliana* codant pour la L-aspartate-oxydase (L-AO) (SEQ ID N°1) a été amplifié par PCR avec les amorces de séquences suivantes :
amorce sens (GAG AGA CCC GGG ATG GCG GCT CAT GTT TCT AC) ;
amorce reverse (GAG AGA CAG CTG AAT CGT TAG TTA TTC ACT CGA C);

L'amplifiat a ensuite été sous-cloné entre les sites Sma11 et Sal1 du vecteur pCW162 binaire, sous le contrôle du promoteur CaMV35S pour générer des plantes transgéniques sur-exprimant la L-aspartate oxydase. La cassette nptll de pCW162 a été utilisée pour la sélection des plantes transgéniques sur milieu contenant de la kanamycine. Le plasmide résultant a ensuite été utilisé pour la transformation stable de plantes d'*Arabidopsis thaliana* pour surexprimer le gène L-AO, en utilisant la souche d'*Agrobacterium tumefaciens* GV3101. Les transformants primaires ont été sélectionnés sur milieu Murashige et Skoog contenant 50 mg.l⁻¹ de monosulfate de kanamycine. Après environs 10 jours de culture *in vitro* (23°C, sous intensité lumineuse de 100 µmoles de photons.m⁻².s⁻¹), les plants résistants ont été transférés sur pot de terreau en chambre de culture jours longs (LD, régime jour/nuit de 16h/8h) afin d'obtenir des graines qui ont subi un nouveau régime de sélection. Le nombre de plantes transgéniques putatives a été noté pour sélectionner les lignées qui ont inséré une seule copie du transgène (rapport ¾ non-résistants, ¼ résistants). Les descendants ont été sélectionnés jusqu'à obtention de lignées stables homozygotes pour l'insertion ADN-T.

### Mesure des niveaux de transcrits L-aspartate oxydase

Après une extraction de l'ARN total en utilisant le kit NucleoSpin RNA II (Macherey- Nagel) selon les instructions du fournisseur, 1 µg d'ARN total a été utilisé comme matrice pour la synthèse d'ADNc premier brin et une transcription inverse avec le système de synthèse du premier brin SuperScript III (Invitrogen). La surexpression du gène de la L-aspartate oxydase a été examinée par RT-PCR avec les amorces des séquences suivantes :
amorce sens (GAT CGT TCA CCG TGC TGA TA) et
amorce reverse (TGT GTT CAA GCC ATC CTG AG)

La lignée, ou plante, contrôle (ctrl) a été obtenue à partir de l'écotype Columbia (Col 0) transformée avec le vecteur pCW1628 vide.

### Culture des plantes

Les lignées transgéniques *d'Arabidopsis thaliana* utilisées dans cette étude ont été obtenues à partir de plantes *d'Arabidopsis thaliana* de l'écotype Columbia (Col 0). Après 48h de stratification à 4°C dans l'obscurité, les graines ont été semées, et cultivées dans des conditions de jours courts (SD, régime jour/nuit de 8 h/16 h), dans une chambre de culture sous un éclairement énergétique de 100 µmol photons.m⁻².s¹ au niveau de la feuille, à 18-20°C et 65% d'humidité, (sauf pour la détermination du nombre de siliques et la mesure de la quantité de semences, il s'agissait de conditions de jours longs, LD, régime jour/nuit de 16h/8h). Un apport de solution nutritive a été réalisé deux fois par semaine.

### Prélèvement des échantillons pour les analyses métaboliques

Des échantillons de feuilles ont été prélevés dans le milieu de la photopériode, congelés rapidement dans l'azote liquide et stockés à -80°C jusqu'à analyse ultérieure. Pour les analyses métabolomiques et transcriptomiques, les plantes ont été analysées et échantillonnées à l'âge de 6 semaines (SD), et à l'âge de 8 semaines (SD) pour l'analyse des échanges gazeux.

### Dosage de l'activité de la L-aspartate oxydase

Une méthode de dosage de l'activité de la L-aspartate oxydase a été développée en utilisant un spectrophotomètre : 0,5 g d'échantillon de feuilles congelées a été broyé dans l'azote liquide, et repris dans 2 ml de tampon d'extraction (Tris-HCl pH 8). Après centrifugation, l'extrait brut a été dessalé par chromatographie d'exclusion de taille sur colonne PD10. Pour 0,7 ml de l'extrait déssalé, 100 µL de L-aspartate 10 mM, 100 µL de fumarate 10 mM et 100 µL de FAD 200 µM ont été ajoutés pour démarrer la réaction, qui a été suivie à 30°C pendant 30 min. La réaction a été arrêtée par chauffage à 100°C 2min pour précipiter les protéines, suivit d'une centrifugation. A 1 mL de surnageant réactionnel ont été ajoutés successivement :
- 0,5 mL de phénolate de sodium 0,33 M pH 13 ;
- 0,5 mL de nitroprussiate de sodium 0,1% ;
- 0,5 mL NaClO 0,2%

L'activité L-aspartate oxydase a été mesurée en spectrophotométrie à DO 635 nm par dosage, contre une gamme étalon de 0 à 100 nmoles de (NH₄)SO₄, du NH₄⁺ provenant de la dégradation quasi instantanée à pH8 de l'iminoaspartate formé au cours de la réaction.

### Mesures métabolomiques

Les dosages de métabolites aux propriétés antioxydantes comme les nucléotides à pyridine NAD⁺ et NADH ont été réalisés par spectrophotométrie via un couplage enzymatique en lecteur de microplaques. Ces métabolites ont été quantifiés par une réaction de recyclage en suivant la réduction du DCPIP (2,6-dichlorophénol-indophénol) à 600 nm en présence d'alcool déshydrogénase et d'éthanol. Le NAD⁺ est dosé après extraction acide : Environ 100 mg de feuilles ont été broyés au mortier dans l'azote liquide, auxquels 1 mL de HCl 0,2 N est ajouté. Après décongélation du broyât, celui-ci a été transféré dans un tube Eppendorf de 2 mL. L'extrait est ensuite clarifié par centrifugation pendant 10 minutes à 14000 g, à 4°C. 200 µL de surnageant ont été chauffés pendant 1 minute à 100 °C, puis neutralisés en ajoutant 20 µL de NaH₂PO₄ 200 mM pH 5,6, et un volume suffisant de NaOH 0.2 M (environ 200 µL) pour atteindre un pH 7. Pour le dosage du NADH, une extraction alcaline a été nécessaire. De la même manière que pour l'extraction acide, 100 mg de feuilles ont été broyés au mortier dans l'azote liquide puis 1 mL de NaOH 0,2 M a été ajouté. Le mélange a ensuite été centrifugé 10 minutes à 14000 g à 4°C. 200 µL de surnageant ont été chauffés pendant 1 minute à 100 °C, puis neutralisés en ajoutant 20 µL de NaH2PO4 200 mM pH 5,6, et un volume suffisant de HCl 0,2 N (environ 150 µL) pour atteindre un pH 7.

La mesure spectrophotométrique des métabolites extraits s'effectue comme suit : Dans chaque puits de mesure ont été ajoutés successivement 100 µL de tampon HEPES 100 mM/EDTA 2 mM pH 7,5, 20 µL de DCPIP 1,2 mM, 10 µL de PMS (phénazine méthosulfate) 10 mM et 10 µL d'ADH (25 U) dans un volume final de 200 µL. Pour les échantillons à tester, 20 µL d'extrait et 25 µL d'eau bidistillée sont ajoutés. Après agitation de la plaque, la réaction est initiée par ajout de 15 µL d'EtOH absolu. Les mesures de NAD sont réalisées à 600 nm par le lecteur de microplaque par référence à une gamme étalon de NAD⁺ ou de NADH.

Le dosage de l'ATP a été réalisé en utilisant le kit ENLITEN ATP Assay System Bioluminescence (Promega) en suivant la procédure recommandée par le fournisseur.

### Mesures d'échange de gaz

Les mesures d'échanges gazeux et de fluorescence de la chlorophylle ont été effectuées en utilisant le système de Li- 6400 xt (Li -Cor, Lincoln, NE, USA) et les paramètres ont été calculés avec le logiciel fourni par le fabricant. Les conditions étaient : une densité du flux de photons ¼ 1.000 mmol m⁻².s⁻¹, une température de la chambre 22°C, un débit de 100 mmol.s⁻¹, une humidité relative de 60 %. Les réponses de l'assimilation nette de carbone (An) et la fraction molaire de CO₂ interne (courbes An/Ci) réalisées dans des conditions ambiantes de teneur en oxygène (21%) ont été mesurées sur les feuilles attachées avec un système d'analyse des gaz infrarouge équipé d'une chambre fluorimètre (Li -Cor 6400-40 ; Li -Cor Inc., Lincoln, Nebraska, États-Unis).

### Test de germination

Afin de s'assurer que les différences observées dans les taux de germination ne sont pas dues à la qualité des semences, les plantes sauvages et des plantes mutantes ont été cultivées côte à côte dans des conditions identiques dans une chambre de culture pour produire des graines fraîches dans des conditions de jours longs. Les graines à pleine maturité et stérilisés ont été semées sur plaques de milieu ¼ Hoagland's, dépourvu de nitrates (0,2 mM) ou riche en nitrates (2,25 mM). Après stratification pendant 2 jours à 4°C dans l'obscurité, les graines ont été placées dans une chambre de culture à 23°C. La protrusion de la radicule a été utilisée comme critère pour l'évaluation des différences de germination entre les types sauvages et mutants de graines.

### est de résistance à des conditions de stress abiotique

Des plantes âgées de 7 semaines cultivées en conditions de jours courts (SD) ont été transférées pendant une semaine en conditions de lumière continue de 350 µmol photons.m⁻².s⁻¹ à 37°C et 65% d'humidité.

### Test de résistance à un stress biotique

Des pucerons *Myzus persicae* issus d'une même colonie maintenue au laboratoire sur des plantes sauvages *d'Arabidopsis thaliana* de même écotype que celui des plantes sauvages et mutantes testées, ont été collectés et transférés sur des plantes fraîches âgées de 5 semaines. En 2 jours, ils ont donné naissance à des larves. Les pucerons adultes ont été retirés et seules les larves ont été gardées. Cela a permis d'obtenir des pucerons de même âge ± 1 jour. 7 jours plus tard, 3 pucerons ont été transférés sur chacune des plantes de chaque génotype âgées de 18 jours. Après 5 jours, le nombre de pucerons a été compté à la loupe pour chaque plante. Par analyse statistique (ANOVA), il a été vérifié que le diamètre des rosettes n'influait pas sur la prolifération des pucerons.

### Analyses statistiques

Sauf indication contraire, les données sont les moyennes et les écarts standards de trois à cinq échantillons indépendants de différentes plantes ; des différences significatives sont exprimées à l'aide du test t de Student à P <0,05. Toutes les expériences ont été répétées au moins trois fois et ont donné des résultats similaires. Le test t de Student et l'analyse de variance à deux voies (ANOVA) ont été mis en œuvre en utilisant le logiciel Excel (Microsoft).

### RESULTATS

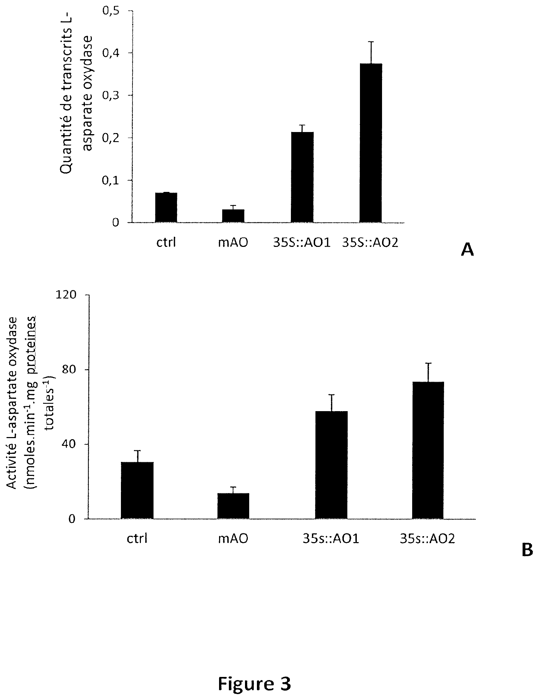

Les résultats indiquent que la séquence nucléotidique d'*Arabidopsis thaliana* utilisée pour la transformation des plantes et qui est homologue de celle de la L-aspartate oxydase bactérienne caractérisée dans la littérature, correspond bien à une activité L-aspartate oxydase et que les lignées transformées présentent une activité de la L-aspartate oxydase augmentée de 2 à 4 fois (figure 3).

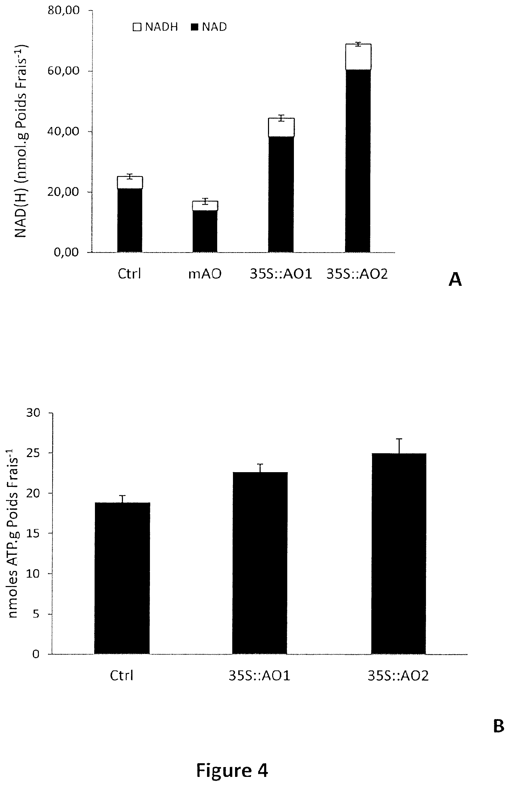

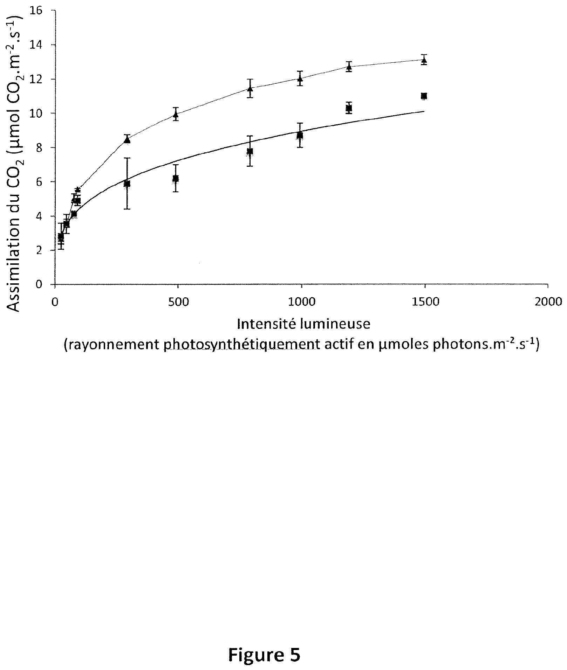

Les résultats démontrent que la surexpression constitutive de l'ADNc de la L-aspartate oxydase entraîne bien une augmentation des niveaux de NAD et des métabolites énergétiques connexes (figure 4).
Les résultats montrent les lignées surexprimant la L-aspartate oxydase présentent des taux d'assimilation photosynthétiques du CO₂ plus élevés (figure 5).

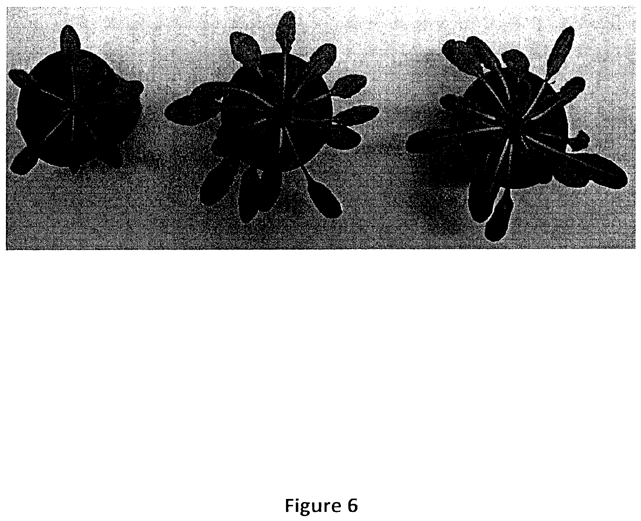

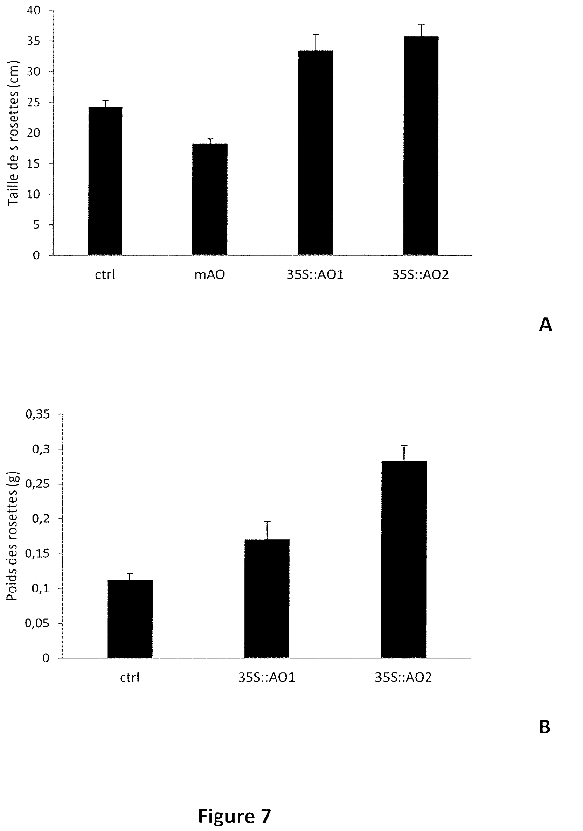

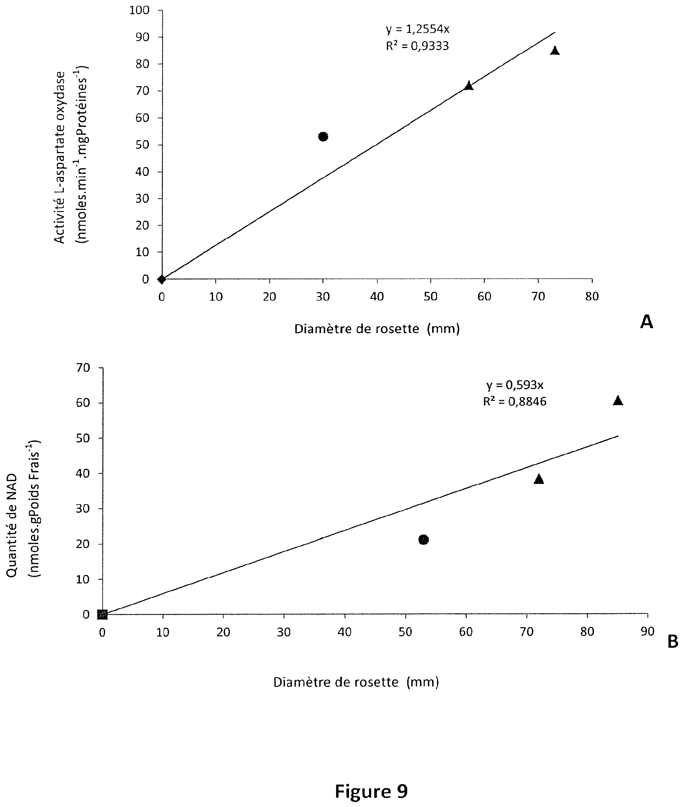

La croissance et le rendement des plantes transformées avec la cassette d'expression de la L-aspartate oxydase ont été évalués. La surexpression de la L-aspartate oxydase conduit à une augmentation de la croissance des racines ainsi que la surface des feuilles. La taille des plantes transgéniques surexprimant la L-aspartate oxydase est supérieure à la taille des plantes témoins du même âge (figures 6 et 7). L'inverse est observé chez des plantes mutantes aux niveaux d'aspartate oxydase fortement réduits (figure 7). Le poids frais des plantules est également augmenté de manière significative (figure 7) et le rapport poids frais/poids sec est inchangé par rapport aux plantes contrôle. En plus de l'augmentation du développement de la plante, une augmentation de la taille moyenne des cellules de l'épiderme peut être détectée. Aucune variation de ploïdie n'a été observée entre les plantes surexprimant et les plantes ne surexprimant pas la L-aspartate oxydase, ce qui confirme que la taille accrue des lignées surexprimant la L-aspartate oxydase n'est pas liée à une augmentation de la ploïdie. Une forte corrélation entre le diamètre de la rosette et l'abondance des transcrits, de l'activité et du niveau de L-aspartate oxydase et de NAD a été observée (figure 9), montrant que l'augmentation de la croissance des plantes dépend directement du niveau d'expression de la L-aspartate oxydase. La surexpression de la L-aspartate oxydase provoque ainsi une augmentation de la croissance cellulaire et une augmentation de la croissance et du développement de la plante entière.

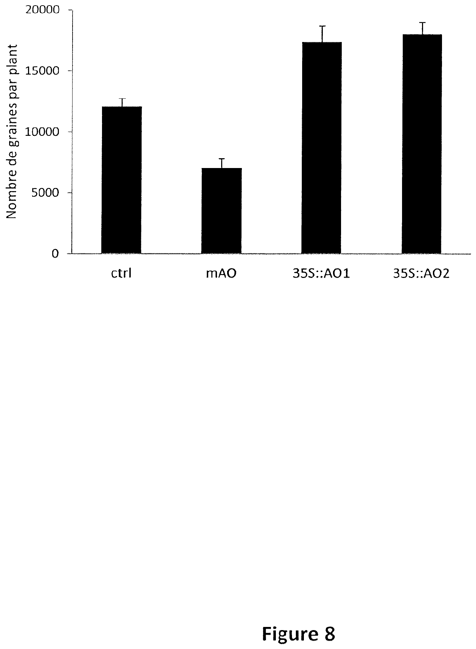

Le rendement en graines des plantes transgéniques surexprimant la L-aspartate oxydase s'est révélé supérieur à celle des plantes contrôle. L'augmentation de 45% du rendement en graines observé (figure 8) est en corrélation avec le nombre de siliques par plante. Cette augmentation de la production de semences ne s'accompagne d'aucun problème de remplissage des siliques dans les plantes surexprimant L-aspartate oxydase par rapport aux plantes contrôle. De plus, la taille des siliques est identique entre toutes les plantes. La surexpression de la L-aspartate oxydase entraîne ainsi un rendement accru en graines. A l'inverse, les plantes mutantes à faible activité L-aspartate-oxydase ont un rendement en graines de 42% inférieur aux lignées contrôle.

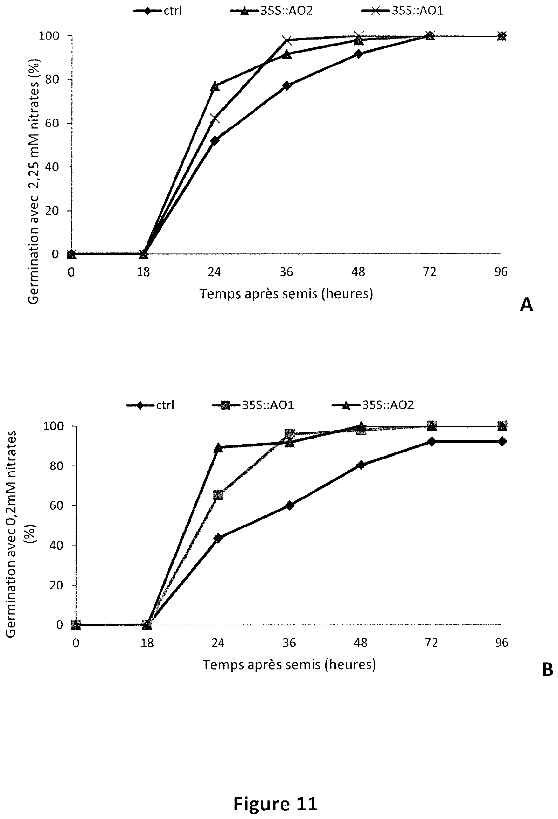

L'augmentation du rendement en semences est concomitante avec une qualité germinative accrue. En effet, les graines produites par les plantes surexprimant la L-aspartate oxydase germent plus vite que les semences témoins (figure 11). La faculté germinative des plantes surexprimant la L-aspartate oxydase n'est pas modifiée dans un environnement pauvre en azote comme il est observé pour les semences témoins (figure 11). La surexpression de la L-aspartate oxydase stimule ainsi la germination et les graines peuvent germer mieux dans des conditions de carence en azote.

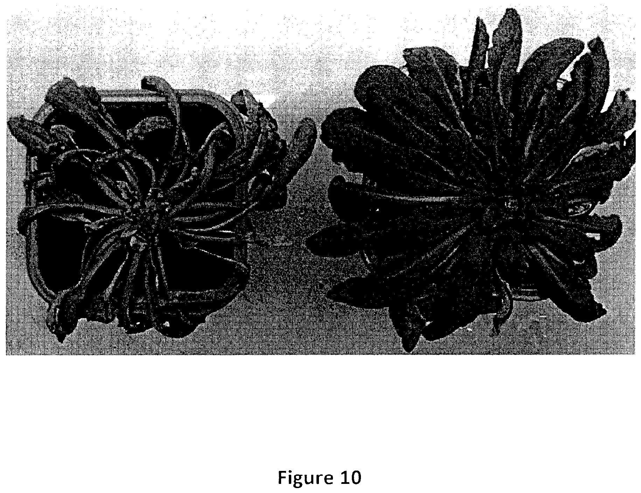

Les plantes surexprimant la L-aspartate oxydase exposées en continu à 350 µmoles photons.m⁻².s⁻¹, et 37°C ont survécu alors que les plantes témoins se dessèchent et meurent dans les mêmes conditions extrêmes (figure 10). La surexpression de la L-aspartate oxydase renforce ainsi la résistance des plantes à des conditions sévères de stress abiotique.

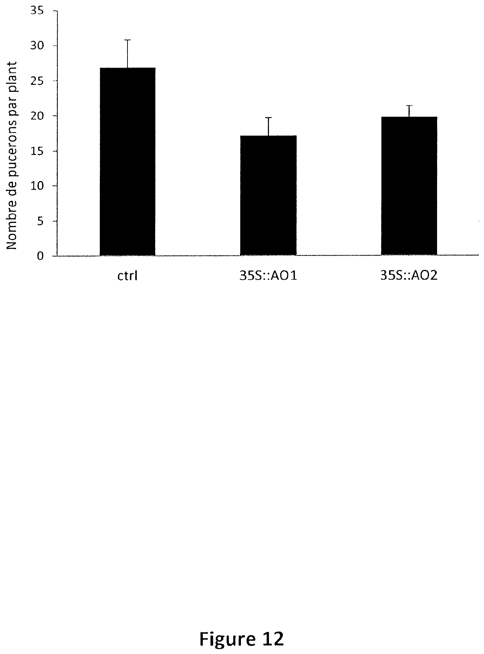

Les plantes surexprimant L-aspartate oxydase cultivées en présence de pucerons *Myzus persicae* ont limité le développement des pucerons par rapport à des plantes témoins infestées dans les mêmes conditions (figure 12). La surexpression de la L-aspartate oxydase renforce ainsi la résistance des plantes à des conditions de stress biotique comme une attaque de pucerons.

Les plantes surexprimant la L-aspartate oxydase exposées en continu à 350 µmoles photons.m⁻².s⁻¹, et 37°C ont survécu alors que les plantes témoins se dessèchent et meurent dans les mêmes conditions extrêmes (figure 10). La surexpression de la L-aspartate oxydase renforce ainsi la résistance des plantes à des conditions sévères de stress abiotique.

Les plantes surexprimant L-aspartate oxydase cultivées en présence de pucerons *Myzus persicae* ont limité le développement des pucerons par rapport à des plantes témoins infestées dans les mêmes conditions (figure 12). La surexpression de la L-aspartate oxydase renforce ainsi la résistance des plantes à des conditions de stress biotique comme une attaque de pucerons.

## Revendications

1. Méthode pour améliorer au moins un caractère phénotypique choisi parmi le rendement en grains, la vitesse de germination ou la croissance chez une plante, ladite méthode comprenant la surexpression de la L-aspartate oxydaseladite méthode comprenant la transformation d'une cellule végétale avec au moins un acide nucléique qui code pour la L-aspartate oxydase, et la génération à partir d'une telle cellule, d'une plante qui surexprime la L-aspartate oxydase.

2. Méthode selon la revendication 1, pour améliorer le rendement en graines, chez une plante, laquelle méthode comprend la transformation d'une cellule végétale avec au moins un acide nucléique qui code pour la L-aspartate oxydase, générant ainsi une plante qui surexprime la L-aspartate oxydase, et la culture de la plante à maturité.

3. Méthode selon la revendication 1, pour améliorer la résistance au stress abiotique et/ou la résistance au stress biotique chez une plante, laquelle méthode comprend la transformation d'une cellule végétale avec au moins un acide nucléique qui code pour la L-aspartate oxydase, générant ainsi une plante qui surexprime la L-aspartate oxydase, et la culture de la plante à maturité.

4. Méthode selon l'une des revendications 1 à 3, dans laquelle le au moins un acide nucléique qui code pour la L-aspartate oxydase est sous le contrôle d'un promoteur assurant la surexpression de la L-aspartate oxydase.

5. Méthode selon l'une des revendications 1 à 4, dans laquelle le au moins un acide nucléique comprend un acide nucléique selon SEQ ID N°1.

6. Méthode selon l'une des revendication 1 à 4, dans laquelle le au moins un acide nucléique comprend un acide nucléique choisi dans le groupe constitué par SEQ ID N°1, SEQ ID N°3, SEQ ID N°4, SEQ ID N°5, SEQ ID N°6, SEQ ID N°7, SEQ ID N°8, SEQ ID N°9, SEQ ID N°10, SEQ ID N°11, SEQ ID N°12, SEQ ID N°13, SEQ ID N°14, SEQ ID N°15, SEQ ID N°16.

7. Méthode selon l'une des revendications 1 à 6, dans laquelle, la plante est choisie dans le groupe constitué par blé, orge, riz, maïs, sorgho, tournesol, colza, soja, coton, pois, haricot, manioc, mangue, banane, pomme de terre, tomate, poivron, melon, courgette, pastèque, laitue, choux, aubergine, peuplier.

8. Méthode selon l'une des revendications 1 à 6, dans laquelle la plante est le riz (*Oryza sativa*)*,* le blé, l'orge ou le maïs.

## Patentansprüche

1. Verfahren zur Verbesserung mindestens einer Phänotyp-Eigenschaft, ausgewählt aus der Kornausbeute, der Keimungsgeschwindigkeit oder des Wachstums bei einer Pflanze, wobei das Verfahren die Überexpression der L-Aspartatoxydase umfasst, wobei das Verfahren die Transformation einer Pflanzenzelle mit mindestens einer Nukleinsäure umfasst, die für die L-Aspartatoxydase codiert, und die Erzeugung, aus einer derartigen Zelle, einer Pflanze, die die L-Aspartatoxydase überexprimiert.

2. Verfahren nach Anspruch 1 zur Verbesserung der Kornausbeute bei einer Pflanze, wobei das Verfahren die Transformation einer Pflanzenzelle mit mindestens einer Nukleinsäure umfasst, die für die L-Aspartatoxydase codiert, wobei somit eine Pflanze erzeugt wird, die die L-Aspartatoxydase überexprimiert, und die Kultur der Pflanze bis zur Reife.

3. Verfahren nach Anspruch 1 zur Verbesserung der Widerstandsfähigkeit gegenüber abiotischem Stress und/oder der Widerstandsfähigkeit gegenüber biotischen Stress bei einer Pflanze, wobei das Verfahren die Transformation einer Pflanzenzelle mit mindestens einer Nukleinsäure umfasst, die für die L-Aspartatoxydase codiert, wodurch somit eine Pflanze erzeugt wird, die die L-Aspartatoxydase überexprimiert, und die Kultur der Pflanze bis zur Reife.

4. Verfahren nach einem der Ansprüche 1 bis 3, wobei die mindestens eine Nukleinsäure, die für die L-Aspartatoxydase codiert, unter der Kontrolle eines Promoters ist, der die Überexprimierung der L-Aspartatoxydase sichert.

5. Verfahren nach einem der Ansprüche 1 bis 4, wobei die mindestens eine Nukleinsäure eine Nukleinsäure gemäß SEQ ID Nr.1 umfasst.

6. Verfahren nach einem der Ansprüche 1 bis 4, wobei die mindestens eine Nukleinsäure eine Nukleinsäure umfasst, die aus der Gruppe ausgewählt ist, die von SEQ ID Nr.1, SEQ ID Nr.3, SEQ ID Nr.4, SEQ ID Nr.5, SEQ ID Nr.6, SEQ ID Nr.7, SEQ ID Nr.8, SEQ ID Nr.9, SEQ ID Nr.10, SEQ ID Nr.11, SEQ ID Nr.12, SEQ ID Nr.13, SEQ ID Nr.14, SEQ ID Nr.15, SEQ ID Nr.16 gebildet ist.

7. Verfahren nach einem der Ansprüche 1 bis 6, wobei die Pflanze aus der Gruppe ausgewählt ist, die von Weizen, Gerste, Reis, Mais, Sorghum, Sonnenblume, Raps, Soja, Baumwolle, Erbse, Bohne, Maniok, Mango, Banane, Kartoffel, Tomate, Paprika, Melone, Zucchini, Kürbis, Salat, Kohl, Aubergine, Pappel gebildet ist.

8. Verfahren nach einem der Ansprüche 1 bis 6, wobei die Pflanze der Reis (*Oryza sativa*)*,* der Weizen, die Gerste oder der Mais ist.

## Claims

1. Method for improving at least one phenotypic trait selected from grain yield, germination rate or growth in a plant, said method comprising overexpression of L-aspartate oxidase, said method comprising transformation of a plant cell with at least one nucleic acid that encodes L-aspartate oxidase, and generation from such a cell of a plant that overexpresses L-aspartate oxidase.

2. Method according to claim 1, for improving the seed yield of a plant, which method comprises transformation of a plant cell with at least one nucleic acid that encodes L-aspartate oxidase, thus generating a plant that overexpresses L-aspartate oxidase, and cultivation of the plant to maturity.

3. Method according to claim 1, for improving the abiotic stress resistance and/or the biotic stress resistance of a plant, which method comprises transformation of a plant cell with at least one nucleic acid that encodes L-aspartate oxidase, thus generating a plant that overexpresses L-aspartate oxidase, and cultivation of the plant to maturity.

4. Method according to one of claims 1 to 3, wherein the at least one nucleic acid that encodes L-aspartate oxidase is under the control of a promoter that overexpresses L-aspartate oxidase.

5. Method according to one of claims 1 to 4, wherein the at least one nucleic acid comprises a nucleic acid according to SEQ ID NO: 1.

6. Method according to one of claims 1 to 4, wherein the at least one nucleic acid comprises a nucleic acid selected from the group consisting of SEQ ID NO: 1, SEQ ID NO: 3, SEQ ID NO: 4, SEQ ID NO: 5, SEQ ID NO: 6, SEQ ID NO: 7, SEQ ID NO: 8, SEQ ID NO: 9, SEQ ID NO: 10, SEQ ID NO: 11, SEQ ID NO: 12, SEQ ID NO: 13, SEQ ID NO: 14, SEQ ID NO: 15, SEQ ID NO: 16.

7. Method according to one of claims 1 to 6, wherein the plant is selected from the group consisting of wheat, barley, rice, maize, sorghum, sunflower, rapeseed, soybean, cotton, pea, common bean, cassava, mango, banana, potato, tomato, pepper, melon, zucchini, watermelon, lettuce, cabbage, eggplant, poplar.

8. Method according to one of claims 1 to 6, wherein the plant is rice (*Oryza sativa*), wheat, barley or maize.


### SEQUENCE LISTING

<110> CENTRE NATIONAL DE LA RECHERCHE SCIENTIFIQUE (CNRS) UNIVERSITE PARIS-SUD 11
<120> PLANTES A RENDEMENT ACCRU ET METHODE D'OBTENTION DE TELLES PLANTES
<130> 367194D33312
<150> FR 1451445
   <151> 2014-02-24
<160> 16
<170> PatentIn version 3.5
<210> 1
   <211> 3912
   <212> DNA
   <213> Arabidopsis thaliana
<400> 1
   atggcggctc atgtttctac tggaaacatt cataatttct atcttgcggg gcaggtttac      60
   aggggacaag ctttttcatg gagttctgct tctactttca tggcaaatcc attcaaagag     120
   ccctcttggt caagtggggt atttaaggct ctaaaagctg agagatgtgg ttgttactct     180
   cgtggtattt cccccatcag tgagacctca aaacctatca gggctgtttc ggtatcttct     240
   tcaacaaagt attatgattt cactgtgatt ggtagtggag tagcgggtct gcgttatgct     300
   ttagaagttg caaagcaagg aacagtcgca gtgattacca aagatgagcc tcatgagagt     360
   aacacaaact atgctcaagg tggtgttagt gctgtgttat gccctttgga ttctgttgaa     420
   agtcatatgc gggacactat ggtcgctggt gctcatcttt gtgatgaaga aaccgtaaga     480
   gttgtgtgta ctgaagggcc tgaaaggatt cgtgaactga ttgcaatggg agcatcattt     540
   gatcacggcg aggatggaaa tttgcatttg gccagagagg gtggtcactc gcattgtagg     600
   atcgttcacg ctgctgatat gacaggaaga gagattgaga gagctttact tgaagctgta     660
   cttaatgatc ccaacatatc tgtcttcaaa caccattttg caatcgatct gctcacttct     720
   caggatggct tgaacacagt ttgtcatggt gtggacactt tgaatatcaa aactaatgag     780
   gtagtacgct ttatatcgaa ggtgacattg cttgcttcag gtggagctgg gcatatctat     840
   ccatcaacca caaatcctct ggtggctact ggagatggga tggcgatggc tcatcgagct     900
   caagctgtga tctcaaatat ggaatttgtg cagtttcatc ctactgccct agccgacgaa     960
   ggtcttccca tcaaactaca aactgctagg gaaaacgcgt tcctcatcac cgaggcggtg    1020
   agaggtgatg gtggcatcct ctataatcta ggaatggagc gattcatgcc tgtttacgat    1080
   gaacgagctg agcttgctcc aagagacgtg gttgcaagaa gtattgatga ccagcttaag    1140
   aaacgaaacg aaaagtatgt gttacttgac ataagccata agccaagaga aaagattctt    1200
   gcccatttcc cgaacatagc ttctgaatgt cttaaacacg gtctggatat cacccgtcag 1260
   cctattccgg ttgtccctgc agcccattac atgtgtggag gagttcgtgc tggtttacaa 1320
   ggcgaaacca atgtccttgg attgtttgta gcaggtgaag tagcatgtac aggcctccac 1380
   ggggcaaatc gtcttgctag taactcgctt ttagaagctc tggttttcgc gagacgggct 1440
   gttcagcctt cgactgagct catgaaacgc acaagacttg atgtatgcgc atcagagaaa 1500
   tggacaaggc ctgttgttgc gacagctaga ttgctaggag atgaagtaat agcaaagatt 1560
   atagctttga ctaaagaagt gagaagagag cttcaggagg taatgtggaa gtatgttggt 1620
   attgtcagat cgacaattcg gctcaccact gctgagagga aaatcgcaga gctagaagca 1680
   aaatgggaaa catttttgtt tgaacatgga tgggaacaaa cagtggtagc tcttgaagct 1740
   tgtgagatga gaaacttgtt ctgttgcgct aagcttgtgg tgagcagcgc gttagctaga 1800
   catgaaagca gaggtcttca ttacatgaca gactttcctt ttgtggaaga aagcaagcgg 1860
   attccgacga ttattctacc gtcttctcct acaacagcta gttggagctc aaggcggtta 1920
   cagaatataa gtagcagctc acttattgat tgctaaatgg cggctcatgt ttctactgga 1980
   aacattcata atttctatct tgcggggcag gtttacaggg gacaagcttt ttcatggagt 2040
   tctgcttcta ctttcatggc aaatccattc aaagagccct cttggtcaag tggggtattt 2100
   aaggctctaa aagctgagag atgtggttgt tactctcgtg gtatttcccc catcagtgag 2160
   acctcaaaac ctatcagggc tgtttcggta tcttcttcaa caaagtatta tgatttcact 2220
   gtgattggta gtggagtagc gggtctgcgt tatgctttag aagttgcaaa gcaaggaaca 2280
   gtcgcagtga ttaccaaaga tgagcctcat gagagtaaca caaactatgc tcaaggtggt 2340
   gttagtgctg tgttatgccc tttggattct gttgaaagtc atatgcggga cactatggtc 2400
   gctggtgctc atctttgtga tgaagaaacc gtaagagttg tgtgtactga agggcctgaa 2460
   aggattcgtg aactgattgc aatgggagca tcatttgatc acggcgagga tggaaatttg 2520
   catttggcca gagagggtgg tcactcgcat tgtaggatcg ttcacgctgc tgatatgaca 2580
   ggaagagaga ttgagagagc tttacttgaa gctgtactta atgatcccaa catatctgtc 2640
   ttcaaacacc attttgcaat cgatctgctc acttctcagg atggcttgaa cacagtttgt 2700
   catggtgtgg acactttgaa tatcaaaact aatgaggtag tacgctttat atcgaaggtg 2760
   acattgcttg cttcaggtgg agctgggcat atctatccat caaccacaaa tcctctggtg 2820
   gctactggag atgggatggc gatggctcat cgagctcaag ctgtgatctc aaatatggaa 2880
   tttgtgcagt ttcatcctac tgccctagcc gacgaaggtc ttcccatcaa actacaaact 2940
   gctagggaaa acgcgttcct catcaccgag gcggtgagag gtgatggtgg catcctctat 3000
   aatctaggaa tggagcgatt catgcctgtt tacgatgaac gagctgagct tgctccaaga 3060
   gacgtggttg caagaagtat tgatgaccag cttaagaaac gaaacgaaaa gtatgtgtta 3120
   cttgacataa gccataagcc aagagaaaag attcttgccc atttcccgaa catagcttct 3180
   gaatgtctta aacacggtct ggatatcacc cgtcagccta ttccggttgt ccctgcagcc 3240
   cattacatgt gtggaggagt tcgtgctggt ttacaaggcg aaaccaatgt ccttggattg 3300
   tttgtagcag gtgaagtagc atgtacaggc ctccacgggg caaatcgtct tgctagtaac 3360
   tcgcttttag aagctctggt tttcgcgaga cgggctgttc agccttcgac tgagctcatg 3420
   aaacgcacaa gacttgatgt atgcgcatca gagaaatgga caaggcctgt tgttgcgaca 3480
   gctagattgc taggagatga agtaatagca aagattatag ctttgactaa agaagtgaga 3540
   agagagcttc aggaggtaat gtggaagtat gttggtattg tcagatcgac aattcggctc 3600
   accactgctg agaggaaaat cgcagagcta gaagcaaaat gggaaacatt tttgtttgaa 3660
   catggatggg aacaaacagt ggtagctctt gaagcttgtg agatgagaaa cttgttctgt 3720
   tgcgctaagc ttgtggtgag cagcgcgtta gctagacatg aaagcagagg tcttcattac 3780
   atgacagact ttccttttgt ggaagaaagc aagcggattc cgacgattat tctaccgtct 3840
   tctcctacaa cagctagttg gagctcaagg cggttacaga atataagtag cagctcactt 3900
   attgattgct aa 3912
<210> 2
   <211> 651
   <212> PRT
   <213> Arabidopsis thaliana
<400> 2
   Met Ala Ala His Val Ser Thr Gly Asn Ile His Asn Phe Tyr Leu Ala
   1               5                   10                  15
   Gly Gln Val Tyr Arg Gly Gln Ala Phe Ser Trp Ser Ser Ala Ser Thr
               20                  25                  30
   Phe Met Ala Asn Pro Phe Lys Glu Pro Ser Trp Ser Ser Gly Val Phe
           35                  40                  45
   Lys Ala Leu Lys Ala Glu Arg Cys Gly Cys Tyr Ser Arg Gly Ile Ser
       50                  55                  60
   Pro Ile Ser Glu Thr Ser Lys Pro Ile Arg Ala Val Ser Val Ser Ser
   65                  70                  75                  80
   Ser Thr Lys Tyr Tyr Asp Phe Thr Val Ile Gly Ser Gly Val Ala Gly
                   85                  90                  95
   Leu Arg Tyr Ala Leu Glu Val Ala Lys Gln Gly Thr Val Ala Val Ile
               100                 105                 110
   Thr Lys Asp Glu Pro His Glu Ser Asn Thr Asn Tyr Ala Gln Gly Gly
           115                 120                 125
   Val Ser Ala Val Leu Cys Pro Leu Asp Ser Val Glu Ser His Met Arg
       130                 135                 140
   Asp Thr Met Val Ala Gly Ala His Leu Cys Asp Glu Glu Thr Val Arg
   145                 150                 155                 160
   Val Val Cys Thr Glu Gly Pro Glu Arg Ile Arg Glu Leu Ile Ala Met
                   165                 170                 175
   Gly Ala Ser Phe Asp His Gly Glu Asp Gly Asn Leu His Leu Ala Arg
               180                 185                 190
   Glu Gly Gly His Ser His Cys Arg Ile Val His Ala Ala Asp Met Thr
           195                 200                 205
   Gly Arg Glu Ile Glu Arg Ala Leu Leu Glu Ala Val Leu Asn Asp Pro
       210                 215                 220
   Asn Ile Ser Val Phe Lys His His Phe Ala Ile Asp Leu Leu Thr Ser
   225                 230                 235                 240
   Gln Asp Gly Leu Asn Thr Val Cys His Gly Val Asp Thr Leu Asn Ile
                   245                 250                 255
   Lys Thr Asn Glu Val Val Arg Phe Ile Ser Lys Val Thr Leu Leu Ala
               260                 265                 270
   Ser Gly Gly Ala Gly His Ile Tyr Pro Ser Thr Thr Asn Pro Leu Val
           275                 280                 285
   Ala Thr Gly Asp Gly Met Ala Met Ala His Arg Ala Gln Ala Val Ile
       290                 295                 300
   Ser Asn Met Glu Phe Val Gln Phe His Pro Thr Ala Leu Ala Asp Glu
   305                 310                 315                 320
   Gly Leu Pro Ile Lys Leu Gln Thr Ala Arg Glu Asn Ala Phe Leu Ile
                   325                 330                 335
   Thr Glu Ala Val Arg Gly Asp Gly Gly Ile Leu Tyr Asn Leu Gly Met
               340                 345                 350
   Glu Arg Phe Met Pro Val Tyr Asp Glu Arg Ala Glu Leu Ala Pro Arg
               355                 360                 365
   Asp Val Val Ala Arg Ser Ile Asp Asp Gln Leu Lys Lys Arg Asn Glu
       370                 375                 380
   Lys Tyr Val Leu Leu Asp Ile Ser His Lys Pro Arg Glu Lys Ile Leu
   385                 390                 395                 400
   Ala His Phe Pro Asn Ile Ala Ser Glu Cys Leu Lys His Gly Leu Asp
                   405                 410                 415
   Ile Thr Arg Gln Pro Ile Pro Val Val Pro Ala Ala His Tyr Met Cys
               420                 425                 430
   Gly Gly Val Arg Ala Gly Leu Gln Gly Glu Thr Asn Val Leu Gly Leu
           435                 440                 445
   Phe Val Ala Gly Glu Val Ala Cys Thr Gly Leu His Gly Ala Asn Arg
       450                 455                 460
   Leu Ala Ser Asn Ser Leu Leu Glu Ala Leu Val Phe Ala Arg Arg Ala
   465                 470                 475                 480
   Val Gln Pro Ser Thr Glu Leu Met Lys Arg Thr Arg Leu Asp Val Cys
                   485                 490                 495
   Ala Ser Glu Lys Trp Thr Arg Pro Val Val Ala Thr Ala Arg Leu Leu
               500                 505                 510
   Gly Asp Glu Val Ile Ala Lys Ile Ile Ala Leu Thr Lys Glu Val Arg
           515                 520                 525
   Arg Glu Leu Gln Glu Val Met Trp Lys Tyr Val Gly Ile Val Arg Ser
       530                 535                 540
   Thr Ile Arg Leu Thr Thr Ala Glu Arg Lys Ile Ala Glu Leu Glu Ala
   545                 550                 555                 560
   Lys Trp Glu Thr Phe Leu Phe Glu His Gly Trp Glu Gln Thr Val Val
                   565                 570                 575
   Ala Leu Glu Ala Cys Glu Met Arg Asn Leu Phe Cys Cys Ala Lys Leu
               580                 585                 590
   Val Val Ser Ser Ala Leu Ala Arg His Glu Ser Arg Gly Leu His Tyr
           595                 600                 605
   Met Thr Asp Phe Pro Phe Val Glu Glu Ser Lys Arg Ile Pro Thr Ile
       610                 615                 620
   Ile Leu Pro Ser Ser Pro Thr Thr Ala Ser Trp Ser Ser Arg Arg Leu
   625                 630                 635                 640
   Gln Asn Ile Ser Ser Ser Ser Leu Ile Asp Cys
                   645                 650
<210> 3
   <211> 1953
   <212> DNA
   <213> Populus tricocarpa
<400> 3
   atgacaacga gtatagctgg tggaagcagc aacctccact accggttaag tttccgccag      60
   gctaaaggtt gtagacaatc ttcttggatt tccagtttga cattcaatgg atgcttgcgg     120
   aatgagattt catggtcgga tgggctatca aagttgttgc agttccagag atataaattt     180
   tcccgatcta ccattagcaa aaactggaaa cctcttggaa caagaaaagt aagtgtatca     240
   tcttgcttga gagatagtac agttaagtac tttgattttg ctgtcattgg aagtggagtt     300
   gctggtctgc gctatgctct tgaagttgca aaatatggaa ctgttgcagt cattaccaag     360
   gctgagcccc atgagagcaa tacaaactat gcccaaggtg gtgttagtgc agtgctgtcc     420
   ccatcagact ctgtggagag tcacatgcag gatactatgg tagcaggtgc ttatctatgt     480
   gatgaggaga ctgtcagagt tgtctgcaca gaaggacctg acagaattag agaattgata     540
   gctatgggtg caatgtttga tcatggggag gatggaaact tgcacctagc aagggaaggg     600
   ggtcactctc accatagaat tgttcatgct gctgatatga ccggaaggga gattgagcga     660
   gctctgctgg aggccgttgt caatgatcct aatatctctg tgtttgagca ccattttgcc     720
   atagatttgc taacttctca ggatggtcct gacatggttt gtcatggtgt ggacactttg     780
   aacactgaaa ctcaacaggt ggttcgattt atttcaaagg tgactttact tgcatcaggt     840
   ggggctgggc atatctaccc atccaccaca aatcctccgg tggcaacagg agatggaatg     900
   gctatggctc accgagctca agctgtgatt tccaacatgg aatttgtgca gttccaccca     960
   actgctttag ctgatgaagg gcttcccata aaaccaatca aagctcgaga aaacgcattt    1020
   ctcatcactg aagctgtaag gggcgatgga ggcatcctct ataacttaga ctgggaaaga    1080
   ttcatgcccc tgtatgatga gagagctgag ctagctccta gggatgtagt ggcgaggagc    1140
   atagatgacc agcttaagaa gcgttgtgag aagtatgtgc tacttgatat tagtcacaag    1200
   cctagagaga agattctctc tcacttcccc aacatagctg ctgagtgcct ccagtatggc    1260
   ctggatataa cccgccaacc aattcctgtg gttcctgctg cccattacat gtgcggtgga    1320
   gtccgtgctg ggcttcaggg ggagacaaat gtgcagggcc tctatgtggc aggtgaggtt    1380
   gcatgcactg gtttgcacgg agcaaaccga ctcgctagca attcattgct ggaagcacta 1440
   gtttttgctc gaagagctgt tcagccatca attgatcaca tgaagagctc tagccttgat 1500
   ctcagtgctt caaattggtg ggccagacca gtagtaccca attcacctgg gagcaatgta 1560
   atggacaacg tatcgaggaa gacaagggaa gtgaggagag agttgcagtc aatcatgtgg 1620
   aagtacgtag ggattgtccg gtcaacaaca aggcttgaaa ccgcggaggg agaaatcagt 1680
   gagttagagg cccagtggga gaagtactta ttcgaggaag gatgggagca gacaatggtg 1740
   gggcttgagg cttgtgaaat gagaaacctc ttttgttgtg caaagctggt agtgagcagt 1800
   gcactcgcta ggcatgaaag ccgtgggctg cactatacga ttgattttcc tcatgtggag 1860
   gaaagtaaga ggctaccaac agttattctt ccatctcttg tgaataataa tacatggagc 1920
   tcacgacagc tacacaagca ggtcattttt tag 1953
<210> 4
   <211> 1929
   <212> DNA
   <213> Solanum lycopersicon
<400> 4
   atggcaactg gtatagcttc aggaagcggg caattatatt tgagggagca tgtctatcgg      60
   aggagtagct atggaaaagc tcattgtcat tccactgtga tcctgagcag catgcaaaac     120
   caaatccatt ggtcttcttg gatttccaaa ctcttacaag ttgatagaag taactattca     180
   caatgtcaag tgaaaacaaa ccggaagtct cacagaggaa caatcaaatc atgccagaga     240
   gaaggctcaa caaggtattt tgattttgct gtgattggta gtggcattgc tggccttcga     300
   tatgctcttg aggttgccaa acatggatct gtggctgtga taaccaaggc cgagcctcat     360
   gagagtaaca ctaactatgc tcaaggtggt gtaagtgctg tgctctgccc taaggattca     420
   gtggagaacc acatgaaaga tacaattgtg gcaggtgctt acctctgtga tgaggagatt     480
   gttagagttg tgtgcactga aggacctgag agaattagag aactgattgc tatgggtgct     540
   tcgttcgatc atggggagga tggcaatctg catctagcca gggaaggggg ccactcccat     600
   cgccgaattg tccatgctgc tgatatgaca ggcagagaga taggaagggc cctattagag     660
   gcggttgtta aggatcctaa tatatatgtg tttcaacacc attttgcaat agatttgttg     720
   accacccagg atggttctga catagtttgt cacggaattg atactataaa cacagaaaca     780
   caggaggtca taagattcat ttcaaaagtg actttgctgg catcaggtgg agctggacat     840
   atctatccaa gcactactaa tccgccagtt gccactggag atggaatagc tatggctcat     900
   cgagctcaag ctgtaatttc caacatggag tttgtgcaat tccacccgac tgccttggct     960
   gatgaaggcc ttcccatcag accatcaaat gccagagaga atgcttttct gataactgaa    1020
   gctgtcagag gtgatggagg cattctttac aacttagata tggagagatt catgccatcg    1080
   tatgatgaaa gagccgagct tgccccaaga gatgtggtag caagaagtat tgatgaccag 1140
   ctcaaaaagc gtggtgaaga gtatgttctt cttgatatca gtcacaagcc caaagagaag 1200
   attctttctc attttcctaa catagctgct gagtgtctcc gctatgggtt agacataaca 1260
   cagcagccga ttccagtggt tcccgcagct cactacatgt gtggtggagt ccgtgctggg 1320
   ctcgaaggtg agactaatgt acgaggtctt tatgtggcag gtgaagttgc atgtactggt 1380
   ttgcatggtg caaaccgact tgctagcaac tccttggctg aagcactagt gtttgcacga 1440
   agagctgtaa agccttcaat agatcacatg aacctttcta aaatcggtca cagtgcttca 1500
   aattggtggc cgcggcctgt agcacccttg ttactaggag atacagtagt taacaaagtc 1560
   attcgtcaga caagggaagt gaggaaagaa ctacagtcaa tcatgtggga atacgttgga 1620
   attgttcggt ctacctcacg actaaccctt gcagaaacca gaatcataga gttggagtta 1680
   aaatgggaac gatacctatt tcagcatggg tgggaaccga ctatggttgg tttagaggct 1740
   tgcgagatga ggaatctctt ctgttgtgcc aagctggttg ttagcagtgc cctttcgcga 1800
   catgagagtc gtgggcttca ttataccatt gacttccctc acgtcgagga aagcaagagg 1860
   ttgcctacag taatttttcc ttcacagcta aatagctcgc ggcaattaca taagcagcag 1920
   atatgttag 1929
<210> 5
   <211> 1944
   <212> DNA
   <213> Glycine max
<400> 5
   atggcaactt gtgttcctgc tgtaagtggt gcactgcatt atggagtgac aaactgcaag      60
   ggacacagct acagaaggag tgcttccttt tctgatgtgc ctaacacggg attcttacaa     120
   aaagatcttt catggtctaa agtggtatcc aaggtcctgc agatacacag atgtgggttt     180
   tctgcacctc cacttcataa ggatcagaaa ctcttcaaag tcatttcctc atggaagaaa     240
   gatagtccca caaaatactt tgactttatc gtcattggga gtggaattgc tggcctccga     300
   tatgcgcttg aagttgcaaa atatggatct gttgcagtga taaccaaggc tgagtctcat     360
   gaatgcaata ctaattatgc tcaaggtggt gttagtgctg tgctatgccc ttcagattct     420
   gtggagagtc acattaagga caccattgtg gctggggcat atctttgtga tgaggaaagc     480
   gtccgggttg tctgcactga aggacccgaa agagtcagag aactgattgc tatgggtgca     540
   tcatttgatc atggggaaga tggtaacttg catctaatga gagagggggg tcattcacat     600
   catagaattg ttcatgctgc tgatatgact ggaaaagaga ttgaacgggc tctactgaag     660
   gaagttatca gtaatcccag aatttttgtg tttgaacacc attttgctat agatcttctc     720
   acttgtcagg atcgatctga tatgatttgt cttggtgttg acacactgaa ttctaaaacc     780
   ctagaggtga ttagatttct ttcaaaggtc actttacttg cgtcaggtgg agctggacat 840
   atttatccta aaactacaaa tcctctggta gccactggag atggaattgc catggcacac 900
   cgagctcaag ctgtgatttc caacatggag tttgtgcagt tccatccaac tgccttagct 960
   gatgaagggc ttcctattaa accaaccaag cctcgggaaa atgcatttct gataactgaa 1020
   gctgtcagag gtgatggggg catcctttat aattttggca tggaaagatt catgcccttg 1080
   tatgatgaga gggcagagct tgctccaagg gatgtggtgg caagaagtat agacgaccaa 1140
   cttaaaaagc gtcatgagaa gtatgtgctc ctcgatataa gtcacaagcc caaggaggaa 1200
   attctctccc atttccccaa cattgcttct acgtgtctcc agtatggttt ggacataact 1260
   cgtcgtccaa tcccagttgt tccagctgct cattacatgt gtggaggagt tcaagctggt 1320
   ctccaaggag agaccaatgt gaaaggtctg tatgtagctg gtgaggtagc atgcacaggt 1380
   ttgcatggag caaatagact tgctagcaac tcattgcttg aggcactggt ttttgcaaga 1440
   agagctgtgc agccctcagt tgatcacatg aagggctcta gccttgatct gactgcatca 1500
   aacttgtggc ctagacccgt tgtgcctttg ccactcggaa gtaatgtcac agacaaaatt 1560
   atgtcaacga caaaggaatt gagggcagaa ctgcaatcca tcatgtggga ctatgtagga 1620
   attgttcggt ccacaatgag actagagact gctgagcaaa aaattggtag tttagaagct 1680
   aaatgggagg agtctctgtt tcagcatgga tggaagccaa caatggtcgg gcctgagatc 1740
   tgtgagatga gaaacctctt ttgttgtgca aagctggtgg tcagcagtgc gctttctagg 1800
   catgagagcc gtggactaca ttacactgtt gattttcctt atctcgagga gagtaagaga 1860
   cttccaacaa tcatttttcc aagttcacct gtaaacagta catggagttc tcggcaatta 1920
   cacaagcagc ccatgtacca gtaa 1944
<210> 6
   <211> 1938
   <212> DNA
   <213> Oriza sativa
<400> 6
   atggcggctc tgatgaacgg ctttgggagc ctccaatgca aggcgacggt gcatgtcgag 60
   aaagggcaca tgcaagcatc ggggatggcc ttcttctctc ccgtcaacag atgcgcccag 120
   gttcatatct ccagtattcc tcatttcatc ggcgcgaagt ctgttagtgc ttcccaactt 180
   cgaatgaggc acaaggtcgg gtccatcaga gcctctgcag cctcgtgctt gcaagatgaa 240
   acaacaaaat atttcgattt tgtggtcatt ggcagcggtg ttgctggcct aaggtatgct 300
   ctggaagtgt ctaagtacgg ctctgtcgcc atcattacca aagcagagcc tcatgagagc 360
   aacacgaact acgcacaagg cggtgtcagc gcggttctat gcccctcaga ttctgtagag 420
   agccacatgc aagacaccat tgtcgcgggg gcttatctgt gtgatgaaga gactgtcagg 480
   gtagtatgca cagaaggacc ggagcgtgtg aaggagctca ttgccatggg agcttcattt 540
   gaccatggcg aggacggaag gctgcacctt gcaagggaag gtggacattc tcacaacaga 600
   atagttcatt ctgctgatat gactggaaga gagatcgaga gagcactgct tcaagcagtt 660
   gataatgatg ataacatatc tttgtttggc caccactttg ccattgattt actcacctgt 720
   cagagtaatg gtgaaatata ttgctatggt gtggattcat tggacgctga aactcagaag 780
   gcaattcgtt tcatctcgaa agtaacattg cttgcatctg gaggagttgg tcatatatac 840
   ccctcaacga ccaatccacc ggtagctact ggggatggaa ttgcaatgtc tcatcgtgcg 900
   caggctgtaa tatctaatat ggagtttgtg cagttccacc caactgcact atcagatgag 960
   ggtctcccaa taaaaccagc tacaagaaga gagaatgcat tcctcataac agaagcagtc 1020
   agaggagatg gaggaattct ttataatcag tccatggaga ggtttatgac ttcgtatgat 1080
   gaccgtgcag agttagcacc gagagatgtg gtcgcaagaa gcatagatga ccaactgaag 1140
   aagaggggag aaaagtatgt cctcctggat atcagtcaca agccaaggga aaaggttctt 1200
   gcccattttc caaacattgc agctgaatgc ctgcgccacg gtctcgacat cacacaacag 1260
   ccgatacctg ttgttcctgc agctcattac atgtgtgggg gtgtccgggc tgggttgcag 1320
   ggggagacaa atgtgaaagg cctgtatgtt gctggtgagg ttgcatgcac tggattgcac 1380
   ggtgctaatc ggcttgcaag caactcactg cttgaagcac tggtgtttgc tcggagagca 1440
   gtgcagcctt caatagatca tatggtcgat gcagatgtcg atccttcttt cgcgaagaaa 1500
   tgggctcgtc ctgtgctgtc tgtctccctt agggacagta tactatctga tatcattgag 1560
   aagacaaagc aggccaggat ggagcttcag tcaataatgt gggagtatgt cgggatagtg 1620
   cggtcgacga accgcctgaa gcacgcggaa tggaagatca gtgatctgga gtcagaatgg 1680
   gaggagttct tgttcaggag ggggtggaag cctacaatgg tgggggttga gacctgtgaa 1740
   atgaggaacc tcttctgctg cgcaaagctg gttgtgaaaa gtgctcttgc aaggcacgag 1800
   agccgaggct tgcacttcac cgaagacttc ccttacctgg aggagagcaa gaggaagcct 1860
   acagtgatct tccctactca tatccaggag ctgacatgga gctcaaagcc attgcagaag 1920
   cagctgcagt gcaagtag 1938
<210> 7
   <211> 1995
   <212> DNA
   <213> Zea mays
<400> 7
   atggcgaacc tgacgaacgg ctttgcgagc ctccattgcg caggggcgat gcatgtggac        60
   aaagggcaca tgcaagcatc gggactgcct tttctctctt tcagaagatg cgcccagctt       120
   gatatctcca gactaggtag cgtgcctcgt ttcatgggcg caacatctgc tactgtctcc       180
   caacatcatg tgaggcagag gatcagcgcc atcagagcct ctactctttc ctgcctgcaa 240
   gacgatgcca caaaattctt tgattttgtg gttattggca gcggtgttgc tggcctaagg 300
   tatgctctgg aagtttcaaa gcacggctct gttgctatta tcaccaaggc agagcctcat 360
   gagagcaaca caaactatgc acaaggcggt gttagtgccg ttctatgccc ctcggattct 420
   gtagaaagtc acatgaaaga cacaattgtt gcaggggctt atttgtgcga tgaagagatt 480
   gtcagggtag tttgcacaga aggtccagag cgtgtcaagg aactaattgc catgggtgcc 540
   tcattcgacc atggtgaaga tggtaggctg caccttgcaa gggaaggtgg tcattctcac 600
   aacagaattg tccattctgc cgatatgact ggaagagaga ttgaaagagc actgcttcaa 660
   gcagttcaca atgatgataa catatctttg tttggtcatc actttgctat tgatctattg 720
   acatgtcaga aaaatggtga aatctattgc tatggagtgg attcaataga cattgaaacc 780
   cagaaggtag tccgcttcat ctcgaaagtg acattgcttg catcaggagg agctggccat 840
   atatatccca caaccaccaa tccaccggta gctactgggg acggaatcgc aatgtgtcat 900
   cgtgctcagg ctgtaatatc caatatggag tttgtgcagt tccatccaac tgcactttca 960
   gatgagggcc tgccaataaa gccaaagaca agaagagaga atgcatttct cataacggaa 1020
   gcggtgagag gagacggagg aattctttac aaccagtcca tggagagatt catgccgatg 1080
   tacgatgacc gctcggagct ggcgccgaga gacgtggttg cgaggagcat agacgaccag 1140
   ctgaagaaac gaggcgaaaa gtatgtcctc ctggacatca gccacagacc aagagacaag 1200
   gttcttgccc acttccccaa catcgccgcc gagtgcctgc ggtacggcct ggacatcacc 1260
   cggcagccca tcccggtggt cccggcggcg cactacatgt gcggcggcgt ccgggcaggg 1320
   ctgcaggggg agaccaacgt gaagggcctc tacgtggccg gcgaggtcgc gtgcacgggg 1380
   ctgcacggtg ccaaccggct agcaagcaac tcgctgctgg aagcgctggt gttcgccagg 1440
   agggcggtgc agccgtccat cgacctcatg gtggatgccg acggagatgc cagaccgtcg 1500
   ctggtggcga ggtgggcacg gccgacgctg ccgcggtcgg tgctgggcga cagcgtgctg 1560
   tcggacatcg tggggcggac gaggcaggcc aggatggagc tgcaatcagt gatgtgggag 1620
   tatgtcggca tcgtgcgctc gacggggcgg ctgaagcagg ccgagtggag gatcggtgac 1680
   ctggagtcgg agtgggagga gttcctgttc cggcgggggt ggaagccgac cacggtgggc 1740
   gtcgaggtct gcgagatgcg caacctcttc tgctgcgcca agctcgtcgt caggagcgcg 1800
   ctggccaggc gcgagagccg cggcctgcac ttcaccgagg acttcccgta cctggaggag 1860
   agcaggagga agcctacggt catcttcccg gccgccgtgc aggagctcac gtggagctcc 1920
   aagccgttgc agaggcagct gcaagcagat gacaatgcat gcagttcatc cggctgggcc 1980
   ttggggattc gttaa 1995
<210> 8
   <211> 1965
   <212> DNA
   <213> Sorghum bicolor
<400> 8
   atggcgaacc tgacgaacgg ctttgcgagc ctccattgcg caggggcgat gcatgtggac      60
   aaagggcaca tgcaagcttc gggactgcct tttctctctt tcagaagatg ctccaagctt     120
   gatatctcca gactaggtac cgtgcctcgt ttcatgggcg caaaatctgc tactgtctcc     180
   cagcatcatg tgagacacag gatcagcgcc accagagcct ctactttttc ctgcctgcaa     240
   gatgatacca caaaattctt cgatttcgtg gttattggca gtggtgtcgc tggcctaagg     300
   tatgctctgg aagtttcaaa gcacggctct gttgctatta tcaccaaggc agagccttat     360
   gagagcaaca caaactatgc acaaggcggt gttagcgccg ttctatgccc ctcggattct     420
   gtagaaagtc acatggaaga cacaattgtt gccggggctt atctgtgtga cgaagagacc     480
   gtcagggtag tttgcacaga aggtccagag cgtgtcaagg aactaattgc catgggtgcc     540
   tcattcgacc atggtgaaga cggtaggctg caccttgcaa gggaaggtgg acattctcac     600
   aacaggattg tccattctgc tgatatgact ggaagagaga ttgaaagagc actacttcaa     660
   gcagttgaca atgatgataa catatcgttg tttggtcatc actttgctat tgatctattg     720
   acatgccaga aaaatggtga aatatattgc tatggagtgg attcgataga cattgaaact     780
   cagaaggtag tccgtttcat ctcgaaagtg acactgcttg catcaggagg agttggccat     840
   atatatccca caaccaccaa tccactggta gctacagggg atggaatcgc gatgtctcat     900
   cgtgctcagg ctgtaatatc caatatggag tttgtgcagt tccatccaac tgcactttca     960
   gatgagggcc tgccaatcaa gccaaagaca agaagagaga atgcattcct cataacggaa    1020
   gcggtcagag gagacggagg aattctttac aatcagtcca tggagagatt catgcccatg    1080
   tacgacgatc gcgccgagtt ggcgccaaga gatgtggttg cgaggagcat agatgaccag    1140
   ctgaagaagc gaggcgagaa ctacgtcctc ctggacatca gccacaagcc aagagagaag    1200
   gttcttgctc acttccccaa catcgccgcc gagtgcctgc ggtacggcct ggacatcaca    1260
   cggcagccca tcccggtggt cccggcggcg cactacatgt gcggcggcgt ccgggccggg    1320
   ctgcaggggg agaccaacgt gaagggcctg tacgtcgccg gcgaggtcgc gtgcacgggg    1380
   ctgcacggcg cgaaccggct ggcgagcaac tcgctgctgg aggcgctggt gttcgccagg    1440
   agggcggtgc agccgtccat cgaccacatg ttggacgccg acggcgacgg cgacggcgac    1500
   ggcgacgctt ccctggcggc gagatgggcg cggccgacgc tgccgtggtc gtcgctgggc    1560
   gacggcgcgc tgtcggacat cgtggagcgg acgaggcagg ccaggacgga gctgcagtcg    1620
   gtgatgtggg agtacgtcgg catcgtgcgg tcgacggggc ggctgaagca ggccgagtgg    1680
   aagatcggcg acatggagtc ggagtgggag gagtttttgt tccggcgggg gtggaagccg 1740
   accatggtgg gcatcgaggc ctgcgagatg cgcaacctct tctgctgcgc caagctcgtc 1800
   gtcaagagcg cgctggccag gcgcgagagc cgcggcctgc acttcaccga ggacttcccc 1860
   tacctggagg agagcaggag gaagcccacg gtcatcttcc cggccgccgt gcaggaggtc 1920
   acgtggagct ccaagccgtt gcagaggcag ctgcagtgca agtag 1965
<210> 9
   <211> 2010
   <212> DNA
   <213> Chlamydomonas reinhardtii
<400> 9
   atggcagtcg cgcttcagcg ctctgctgtg ggtaagcgcc tgcgcgccgc taccccggtc      60
   cgcgtcggcc tgagcgagtc ggctcggcga cctgtggcgc gccccactgg tgtggccgct     120
   cgtcgggtca cccgcagctt cgtcgtccgc gctgacgtga actccgatgc gttccagcca     180
   cgcccggcgg gcgccccggc cgcgcgtgtc gcgcccgaga acacgccgcc tgtccgtcaa     240
   cacgatttcc tcgtcatcgg ctcgggcatc gccggcctga cgtacgcgct gaaagtggca     300
   gagtatggca cggttgccat catcaccaag gacaacgctg ctgagggctg cactcggtat     360
   gcgcaaggag gcgtgtgcgc tgtgctggac aagagcgaca gtgttgctga tcacgttcgt     420
   gacaccattg ttgctggcgc cttcttgaac gatcctcagg ccgtggaagt tgtgtgccgt     480
   gagggccccg cccgcgtgct ggagctggtg gagctgggcg ccgagttctc ccgcaacaac     540
   gatggctccc tgcacctgac caaggagggg ggccacagca accgccggat cgtgcacgcc     600
   gcggatatga cgggcgcaga gatcgagcgc gcgctgctga cgtccgccaa gagccaccgc     660
   aacatccact tctatgagca ccacctggcg gttgacctag tcgtggacga gtatggtggt     720
   atgctgcact gcttcggcgc cgacgtgctg gaccagcgtt ccggcacgat gagcaggttc     780
   ctgggcctgg ccacgctgct ggctagcggt ggcgctggtc aggtgtaccc caacaccacc     840
   aacccgcacg tcgccaccgg agacggcatc gctatggcct accgcgcgca cgccaacgtg     900
   agcaacatgg agttcgtgca gttccacccc actggcctgt acaaccccgc cggtggtgag     960
   ggcagcacct tcctgatcac cgaggccgtc cgcggcgagg gcggtatgct gttcaacaag    1020
   gccggcgagc ggtttatgga gacgtacgac aaggagcgcc tggagctggc cccgcgcgac    1080
   gtggtggcac gcgccattca cgaccagatg cgcctgggca acgagcacgt gtggctggac    1140
   atcagccaca agcctcgcga tgaggtgcta caccacttcc ccaacatcgc ggcgcgctgc    1200
   cttaccctgg gtatcgacat cgccgctgac cccatcccgg tggtgccggc gcagcactac    1260
   acgtgcggcg gcgtcaacac gggcctgctg ggcgagacca atgtgcaggg gctgtacgcg    1320
   tgcggcgagg tggcctgctc cggcctgcac ggcgccaacc gcctggcttc caactctctg    1380
   ttggagggcc tggtgtttgc agagcgtgcc gtcaacccca gcgtggcgca cgctgagcac 1440
   gcgctgcgca actgcggccg gcagctgcac tacgccgctg ccagcgcgga tttccgcggc 1500
   gcgcgtggcg cgcgggagct gacgcccgag ctggcgcagt gggtgtcggc gcggcgtcaa 1560
   gagctgcgcg acatcatgtg gcgctactgc ggcatcgtgc gcaggaccaa ggagctgcag 1620
   caggcccgcg acttcgtggt gtcgctgtac attgagacga aggccatcta caagaactac 1680
   ggcgtgaaca cgcagttggt ggagctgctc aacatggcca ccgtggcgga gctgacggtg 1740
   tcgtgcgcgc tgcagcgcaa ggagagccgc ggcctgcact ttagcgccga ctacccgcac 1800
   ctggacgacg cgcagcgccg ccccagcatg atcagcacct cgctcaagac gcgctacgac 1860
   ctgtcgccct acatgcgcaa cgtgccatct gtgctgcccg ccggggctgg cggccccgca 1920
   agcccggcgc agggcaagcg cctggcgccg cgcaagcagc ccacccgcga gcgcgagttg 1980
   gcggtgcgct ccacgccgca agacctgtga 2010
<210> 10
   <211> 2004
   <212> DNA
   <213> Phaseolus vulgaris
<400> 10
   atgcagtcgc ctacttgtat tgcaatggcg acttggatac ctgctggaag tggcacactg      60
   caatttagag tgacaaactg caagggacac agttgtagaa ggactgcttt ggtttctgat     120
   gccccaatcg cgagattctt acgaaaagat ctttcatggt ctaaagcagt atccaaacac     180
   gtttgtgcat tttctgcacc tccacttcaa aagaatcaga aactcttcaa aatcgtttcc     240
   tcttgcaaga tagatagtct cacacagtgc tttgactttt ccattattgg gagtgggatt     300
   gctggtctcc gctatgcgct tgaagttgca aaatatggat ctgttgcagt gataaccaag     360
   gctgagtctc acgaatgcaa tactaattat gctcaaggtg gtgttagtgc tgtgctaggc     420
   ccttcagatt ctgtggagag tcacatgaag gacaccattg tagctggggc atatctttgt     480
   gatgaggaaa gcgtccgagt tgtctgtact gaaggacctg aaagagtcag agaattaatt     540
   gctatgggtg catcatttga ccatggggaa gatggtaact tgcatcttat gagggaggga     600
   ggtcattcac atcatagaat tgttcatgct gctgatatga ctggaaagga gattgaacgt     660
   gctctactga aggcagttat caacaatcct aatatttttg tgtttgaaca ccattttgct     720
   attgatcttc ttacttgtca ggatgaatct gacataattt gtcttggtgt tgacacgctg     780
   aacactaaaa ccctagaggt gggaacagca gtgataagat ttctttcaaa ggcgacttta     840
   cttgcatctg gtggagcagg gcatatttat cccaaaacta caaatcctct ggtagccact     900
   ggagatggaa ttgccatggc tcatcgagct caagctttga tttccaacat ggagtttgtt     960
   cagttccatc caactgcctt agctgatgaa gggcttccta tcaaacaaac caagcctagg    1020
   gaaaaagcat ttctgatatc cgaagcggtt agaggtgatg ggggcatcct ttataattta 1080
   ggcatggaaa gattcatgcc cttgtatgat gagagggcag agcttgctcc aagggatgtt 1140
   gtggctagaa gcatagatga ccaacttaaa aagcgtgatg agaagtatgt tcttcttgat 1200
   ataagtcaca agccgaagga ggaaatcctc tcccattttc ccaacatttc atctacgtgt 1260
   ctcaagtatg gtttggacat aactcgtcac ccgatcccgg ttgttccagc tgctcattac 1320
   atgtgtggag gagttcaagc tggtcttcaa ggagagacca atgtgaaagg tctgtatgta 1380
   gctggtgagg tagcatgcac aggtttgcac ggagcaaaca gacttgctag caactcattg 1440
   cttgaggcac tcgtttttgc aagaagagct gtgcagccct cagttgattg gatgaagagc 1500
   tctagccttg atctgactgc atcaaacttg tggcctcgac ctgctgctgc gcctttgtcg 1560
   ctcgaaagta atgtcactga caagattctg tcaatgacaa aggaatcgag gacagaactg 1620
   caatccatca tgtggaacta tgtaggaatt gttcggtcga cgatgagact agagacagca 1680
   aagcaaaaca tttgcaactt ggaggctaaa tgggaggagt gcttgtttga gcatggatgg 1740
   aagccaacaa tggcaggtcc tgagatctgt gagatgagaa acctcttttg ctgcgcaaag 1800
   ctggtgatca gcagtgctct ttcgaggcac gagagtcgag gactgcatta cacagttgat 1860
   tttcctcatc ttgaggaaag caagagactt ccaacaatca tttttccaag ttcaactgta 1920
   aacagtacat ggagttctcg acaattacac aagcagccca tgtaccagga aggcatcaaa 1980
   ttgtgtcaca atacatataa atag 2004
<210> 11
   <211> 1863
   <212> DNA
   <213> Physcomitrella patens
<400> 11
   atgttttttt tgaacgctat tccgataaat ctctgtggtg ttttcttgca gctaaacggg 60
   cgggacagga gaaatgaaat tagaaagttt attgccgcct cgaatcgtaa catccaggca 120
   gaagcgagct tatctggtga tggcgaagag gaggagatca cgaaatactt cgatttcctc 180
   gtaatcggaa gtggaatagc aggcttgaga tatgctctgg aagttgccaa gtacggttca 240
   gttgcaatac tcacgaagtc cgaaccgcac gagagtaaca ctgtgtatgc acagggaggt 300
   gttagtgctg ttttagatcc cgaagattct gtagagagtc acatccgaga cacgatcgtc 360
   gctggtgctt tcttatgtga tgaggagact gtagaggtcg tgtgtcgaga aggtccagag 420
   cgtgtgaaag agttgatggc ttggggtgca ttgtttgatc aaagtgaaga tggtcagctc 480
   catctagcta gggaaggagg ccactctcat catcgcattg ttcatgcggc tgatatgact 540
   gggcgagaga tcgagagagc tcttttgact accgtttctc aaaatccgga cattgtaatt 600
   tttaaacatc atgctgctgt ggatcttctc actcttcagg acggggacag tgtagtatgc 660
   tatggtgtgg atgctctaga tacgcagaaa aataaagtta tgcgttttct agccggagtt 720
   acgatgttag catcaggagg tgcagggcac gtttatccta ctacgaccaa tcctttggtg 780
   gcaaccggcg atggagttgc tctggcacaa cggtctcgtg cagtggtgtc taacatggaa 840
   tttgtacagt ttcatcccac ggctttggcg gatgaaggct taccaataaa acctgccaaa 900
   cgggacaatg catttctgat tacggaagct gtgcgtggag ctggaggaat tttgtataac 960
   caaagtatgg agagatttat gccattatat gactctcgcg cggagctcgc acctcgggat 1020
   gttgtggccc ggagcattga tgatcaactc aagaaacgta atgagaaatt cgtttatctg 1080
   gatatcagcc ataaaccagc cagcgagata atgtctcact ttccaaatat tgctgctgag 1140
   tgcctgaagt atgggctgga tattacgaag caacccatcc ctgtggtacc tgctgcacat 1200
   tacatgtgtg gaggcgtaca gactggtttg gtgggagaga cctcaatcaa ggggctcttt 1260
   gccgctggtg aagttacttg taccggtttg cacggtgcaa atagacttgc cagcaattcc 1320
   ttactggagg ctttagtgtt tgcggagaga gctgtcgagc cttcagttgc ttacgcactt 1380
   cagagtaaca ttggacgatc ggaggtgaag caagcgaagg agtggcctcg tcctactgct 1440
   ccctcagctt caaatgactt gcagatggat gagattctcc gcattacagc tttgaaaagg 1500
   aagcgattgc aacaagcaat gtgggatttt gtcggaattg ttcggtctac agagcgtctc 1560
   aagcgtgctc aagcggagtt aagggagctg gagtgtgaat gggaggcaca actcctcaga 1620
   catggttgga aacctaccat ggtcagctta gaggtttgtg agatgcgcaa cttggtatcg 1680
   gtgtcctacc tgattgtgaa cagcgctctc acacggcaag agagtcgtgg ccttcattac 1740
   acgacaagct ttccagaact tgttgagagc gaaaggtttc ctaccattct ttgtccgacc 1800
   gctgcaatga acaattcgtg gagttcacag gttgttcaca gattggcaat agaatctcaa 1860
   taa 1863
<210> 12
   <211> 1602
   <212> DNA
   <213> Rhodothermus marinus
<400> 12
   atgcccgagc gttatcagtt cgattttctg gtcatcggta gtggcattgc cggcctgacg        60
   ttcgcgctgc gcgtggccga tcacggctcg gtcgccatcg tcacgaaaaa agaaagcgtc       120
   gagtcgaaca cgaactacgc gcagggcggc attgcggccg tcatggacgc ggccgattcg       180
   ttcgaacagc acgtgcagga tacgctcgaa gccggggccg gcctgtgcga ccgcgaggtg       240
   gtggaaacgg tcgtgcggga agggcccgaa cgggtgcgtg aactgatggc gctcggggca       300
   cagttcaccc gcgaaaacgg ccggctgcat ctggggcgtg agggcggcca ctcccggaat       360
   cgcatcgtgc acgccgccga cgcgaccggc cgcgaggtcg agcgggcgct gctggcgcgc       420
   gtgcgcgccc acccgaacat tcacatcttc gagtaccatt atgcggttga tctgatcacc       480
   gagcatcatc tcggccagta cgtctcgcgg ttgcgtcccg acatccactg cttcggggcg       540
   tacgtgctgg acgagcgggc cgacgtggtg catacgtttc tggcgaaggc caccctgctg       600
   gccacgggcg gttccgggca ggtctacctg cataccacga acccgccggt ggccacgggc       660
   gacggggtgg ccatggccta tcgggccaag gcccgcgtgg ccaacatgga attcattcag       720
   tttcacccga ccacgctttt ctaccccgac ggtcccaagg aacgctcgtt tctgatcagc       780
   gaagcggtgc ggggtgaggg agcccggctc tacaacctgg ccggcgagcg cttcatgccg       840
   aagtatgacc cgcgggccga gctggcgccg cgcgacattg tggcgcgcgc catcgacgac       900
   cagctcaagc ggcgcggcga tccgcacgtc tggctggaca tctcgcaccg gccggccgag       960
   gaaatcaaac gccgcttccc gaacatctac cggacgctgc tcgactacgg catcgacatg      1020
   acacagcagc ccattccggt cgtgccggcc gcgcactacc agtgtggtgg cgtactgacc      1080
   gacctgcacg gtcgcaccac gattcatggt ctgtacgcag ccggcgaggt agcctgtacg      1140
   ggactgcacg gcgccaaccg actggccagc aactcgctgc tggaggcgct cgtctttgcc      1200
   cgacgggcgg ccgaagacgc cgtgcagtac atccagacgc agacgtggcg tacggacgtg      1260
   ccggactggg acgaccgggg caccgagcgc ccgcaggaat gggtgctcat cgcgcacaac      1320
   cgggacgagc tgcggcgcat catgtgggac tacgtgggca tcgtgcgctc gcagcttcgt      1380
   ctggagcggg ccctgcgccg cacccgactg ctctacgaag aaaccgagga cttctaccgt      1440
   cgcgcccgac tctcgccggg gctgtgtgaa ctgcgtaaca tgatcgccgt ggcttacctg      1500
   atcattcgca gcgctctgat gcgccgcgag agccgcggct tgcactacat gctcgactat      1560
   ccggagcccg tcgagagcga gcgccggccc acgctggtct ga                         1602
<210> 13
   <211> 1623
   <212> DNA
   <213> Escherichia coli
<400> 13
   atgaatactc tccctgaaca ttcatgtgac gtgttgatta tcggtagcgg cgcagccgga      60
   ctttcactgg cgctgcgcct ggctgaccag catcaggtca tcgttctaag taaaggcccg     120
   gtaacggaag gttcaacatt ttatgcccag ggcggtattg ccgccgtgtt tgatgaaact     180
   gacagcattg actcgcatgt ggaagacaca ttgattgccg gggctggtat ttgcgatcgc     240
   catgcagttg aatttgtcgc cagtaatgca cgatcctgtg tgcaatggct aatcgaccag     300
   ggggtgttgt ttgataccca cattcagccg aatggcgaag aaagctacca tctgactcgt     360
   gaaggtggac atagtcaccg tcgtattctt catgccgccg acgccaccgg tagagaagta     420
   gaaaccacgc tggtgggcaa ggcgcagaac catccgaata ttcgcgtgct ggagcgcagc     480
   aacgcggttg atctgattgt ttctgacaaa attggcctgc cgggcacgcg atgggttgtt     540
   ggcgcgtggg tatggaaccg aaataaagaa acggttgaaa cctgccacgc aaaagcagtg     600
   gtactggcaa ccggcggtgc ttcaaaagtt tatcagtaca ccaccaatcc ggatatttct     660
   tctggcgatg gcattgctat ggcgtggcgc gcaggctgcc gggttgccaa tctcgaattt     720
   aatcagttcc accctaccgc gctgtatcac ccacaggcac gcaatttcct gttaacggaa     780
   gcactgcgcg gcgaaggcgc ttatctcaag cgcccggacg gcacgcgttt tatgcccgat     840
   tttgatgagc gcggcgaact ggccccgcgc gatattgtcg cccgcgccat tgaccatgaa     900
   atgaaacgcc tcggcgcaga ttgtatgttc ctcgacatca gccataagcc cgccgatttt     960
   attcgccagc atttcccgat gatttatgaa aaattgctcg ggctggggat agatctcacg    1020
   aaagaacccg tgccgattgt gcctgctgca cattatacct gcggtggtgt aatggttgat    1080
   gatcatgggc gtacggacgt cgatggtttg tatgccattg gcgaagtgag ttataccggc    1140
   ttacacggcg ctaaccgcat ggcctcgaat tcattgctgg agtgtctggt ctatggctgg    1200
   tcggcggcag aagatatcag cagacgtata ccttatgccc acggcgtcag tacgttaccg    1260
   ccgtgggatg aaagccgcgt tgaaaaccct gacgaacggg tagtaattca gcataactgg    1320
   cacgagctac gtctgtttat gtgggattac gttggcattg ttcgcacaac gaagcgcctg    1380
   gcacgcgccc tgcggcggat aaccatgctc caacaagaaa tagacgaata ttacgcccat    1440
   ttccgcgtct caaataattt gctggagctg cgtaatctgg tacaggttgc cgagttgatt    1500
   gttcgctgtg cgatgatgcg caaggagagc cgtggcctgc atttcacgct ggattatccg    1560
   gaactgctca cgcattccgg tccatcgatc ctttctgccg gcaatcatta cataaacaga    1620
   taa                                                                  1623
<210> 14
   <211> 1596
   <212> DNA
   <213> Bacillus subtilis
<400> 14
   atgtctaaaa agacgattgc agtcatcggt tcaggggcgg cggcactttc cttagctgca      60
   gctttcccgc cctcatacga agtgactgtt atcacaaaaa aaagcgtcaa aaacagcaat     120
   tctgtctatg cacaaggcgg gattgctgcg gcctatgcaa aagatgactc gattgaagcc     180
   catctggagg atacgttata cgcagggtgc ggccataata atctagctat tgtagcagac     240
   gtattacatg atggaaaaat gatggttcaa agcctattgg agcgtggatt tccattcgac     300
   cgcaatgaac gaggcggtgt ttgtcttgga agagaagggg cccactcata caaccgcata     360
   tttcatgcag gcggagacgc aacaggcagg ctgcttatcg attatttgct caaacggatc     420
   aacagcaaaa tcaagttaat cgagaatgaa acggcagcag atttgcttat agaggacgga     480
   cgatgtatcg gcgtcatgac aaaagacagc aaagggcggc tcaaggtaag gcacgcagac     540
   gaagttgtgc tggctgccgg cggctgtgga aacctgtttc ttcaccatac gaatgatctc     600
   actgtcacag gtgacggtct ttctctggct taccgggccg gggcggaatt aacggatttg      660
   gagtttactc agtttcaccc gacactgctt gtgaaaaatg gtgtttccta cgggcttgtt     720
   tcagaagctg tcagagggga aggcggatgt ttagtagacg aaaacggccg caggattatg     780
   gctgaacggc atcctttagg tgacctggct ccaagagata ttgtctcacg ggttattcac     840
   gaagaaatgg caaaaggaaa tcgcgtttat atagacttca gtgcgatttc tgattttgaa     900
   acgcgtttcc ctaccatcac cgctatttgt gaaaaagcag ggattgatat ccacagcgga     960
   aaaattcctg ttgctccggg aatgcatttc ttaatgggag gtgtttcagt taatcgctgg    1020
   ggagaaacga cagtgccggg gctttatgcg attggcgaga cagcatgctc cggtttacat    1080
   ggagcaaacc ggcttgcaag caattcttta ttggaagcgc tggtatttgg aaaaagggca    1140
   gcagagcata ttatccaaaa accggtttat aacaggcaat atcaatcagg gctagaaacc    1200
   agtgtcttct acgaggtgcc ggatatagag gggcatgaac tgcaaagcaa aatgacaagc    1260
   cacatgtcta ttctgcggga acaaagcagt ttgattgagc ttagcatctg gcttcatacg    1320
   ctgccttttc aggaagtaaa tgtgaaggat atcacaattc ggcagatgga actttctcat    1380
   ctatggcaaa ctgcaaagct gatgacgttt tctgcgcttt tgcgggagga aagcagaggg    1440
   gctcacttcc gcaccgattt tcctcatgct gaggtgagct ggcaaggaag acaaattgtc    1500
   catacaaaaa aaggaacgaa gatcagaaaa aacgagggga tttggaacaa tgaatcattt    1560
   acagctgaaa aaattactga atcacttttt tcttga                              1596
<210> 15
   <211> 1413
   <212> DNA
   <213> Triticum aestivum
<400> 15
   atgggtgctt catttgatca tggtgaagat ggcaggctcc accttgcaag ggaaggtgga      60
   cattctcaca acagaatcgt ccattctgcc gatatgactg gaaaagagat tgaaagagcg     120
   ctgcttcaag cggttgaaaa tgatgagaac atatctgtgt tcggccacca cttcgccatt     180
   gatctgttga cctgtcagaa taatggtgaa atcttttgtt acggagtgga ttcattggac     240
   accaaagctc aaaaggtagt ccgtttcatc tcaaaagtaa cattgcttgc gtccggagga     300
   gctggccata tatatcccac aacaaccaat ccaccggtgg ctactgggga tggaatcgca     360
   atgtgccatc gcgctcaggc tgtgatatcc aatatggagt ttgtgcaatt ccatccaact     420
   gcactatcag acgaaggcct tccaataaag ccagctaaaa taagagataa tgcatttctt     480
   gtaacagaag cagtcagagg agatggagga attctttaca accaatccat ggagagattt     540
   atgcctttat acgacgaccg tgccgagttg gcaccgaggg atgtggttgc aagaagcata     600
   gatgatcaac tgaagaaacg tggagagaag tatgttctct tggacatcag ccacaagcca     660
   agggagaaaa ttcttgctca ttttccgaac attgcagctg aatgcctgcg gcacggtctg     720
   gacatcacac agcagcccat acctgtcgtc cctgcagctc attacatgtg cggtggtgtt     780
   cgggctgggt tgcaagggga gacgagtgtg aaaggcttgt atgtcgctgg tgaggttgct     840
   tgcactggat tgcacggtgc taatcgtctt gcaagcaact cattgctgga agcgttggta     900
   tttgctcaga gagccgtgca gccctctatc gaccacatgg tggatgcgga tgctgaccct     960
   tgtctcgcgg agaaatgggc acgccctgtg ctctctgtct ccattaagga cagtgcactg    1020
   tctgacatca ttgagaggac aaagaagacc aggatggagc tgcaatccat aatgtggaag    1080
   tacgttggta tagtgcggtc gacgaaccgg ctgaagaatg cagaatggaa gattggtgat    1140
   ctagagtcag agtgggagga attcttattc aggaggggct ggaagcctgc ctcagtgggg    1200
   atcgaggcct gcgaaatgag gaacctcttc tgctgcgcaa agctggttgt gaagagcgcg    1260
   cttgcgaggc gggagagtcg tggcctgcac ttcactgagg acttccctta cctggaggag    1320
   agcaagagga agcctacagt gatcttccct actgctatcc aagagctaac atggagttca    1380
   aagccattgc agaggcagct gcagtgcaaa tag                                 1413
<210> 16
   <211> 1923
   <212> DNA
   <213> Hordeum vulgare
<400> 16
   atgggcggct ctgctggcct ccaccatgtg catgtgcagg gagcacacat gcaggcgtct      60
   cgcccgcctt ctctctcctt cagagcctgc gcccagctcg agatctccag attttgcact     120
   acccctcgtt tcatgtccgt gaaggctctc agtgcttcgc aacagcatac gaggcacagg     180
   ttcagctcca tcagagcctc tgccctccca tgcatgcagg atgacaccac gagatacttt     240
   gatttcgtgg tcatcggcag tggtgtcgct ggcctaaggt atgcgctaga agtctcgaag     300
   cacggctctg ttgctatcat caccaaagca gagcctcatg agagcaacac caactacgcg     360
   caaggcggcg tcagtgccgt tctgtgcccc aaggattccg tagaaagcca tatgcaagat     420
   actattgttg caggggctta tctctgcgat gaggagaccg tcaggatagt atgcacagaa     480
   ggtcctgagc gtgtcaagga gctaatagcc atgggtgctt catttgacca tggtgaagat     540
   ggcaggctgc acctcgcaag ggaaggtgga cattctcaca acagaatcgt acattctgct     600
   gatatgactg gaaaagagat tgaaagagcg ctccttcaag cggttgaaaa tgatgagaac     660
   atatctgtgt tcggccacca cttcgccatt gatctgttga cctgtcagaa caatggtgaa     720
   atcttttgtt acggagtgga ttcgttggac accaaagctc aaaaggtagt ccgtttcatc     780
   tcaaaagtaa cattgcttgc atctggagga gctggccata tatatcccac aacaaccaat     840
   ccaccggtgg ctactgggga tggaatcgca atgtgtcatc gcgctcaggc tgtgatatcc     900
   aatatggagt ttgtccagtt ccatccaact gcactatcag acgaaggcct cccaataaag     960
   ccagctaaaa caagagataa tgcatttctc gtaacagaag cagtcagagg agatggagga    1020
   attctttaca accaatccat ggagagattt atgcctttat acgacgaccg tgctgagttg    1080
   gcaccaaggg atgtggttgc aagaagcata gatgatcaac tgaagaaacg tggagagaag    1140
   tatgttctgt tggacatcag ccacaagcca agggaaaaaa ttcttgctca ttttcccaac    1200
   attgcagctg aatgcctgct gcacggtctg gacatcacac agcaacccat acctgtcgtc    1260
   cctgcagctc attacatgtg cggtggtgtt cgggctgggt tgcaagggga gacaagtgtg    1320
   agaggcttgt atgtcgctgg tgaggttgct tgcactggat tgcacggtgc taatcgtctt    1380
   gcaagcaact cattgctgga agcattggta ttcgctcaga gagccgtgca gccctctatc    1440
   gaccacatgg tggatgctga tgctgacccg tgtctcgcag agaaatgggc acggcctgtg    1500
   ctctctgtct ccattaggga cagtgcactg tctgacatca ttgagaggac aaagaagacc    1560
   aggatggagc tacaatccat aatgtgggag tatgtcggta tagtgcggtc aacgaaccgg    1620
   ctaaagaatg cagaatggaa gattggtgat ctagagtcag agtgggagga attcttattc    1680
   agggggggct ggaagcctgc cacggtgggg atcgaggcct gcgaaatgag gaacctcttc    1740
   tgctgcgcaa agctggttgt gaagagcgcg cttgcgaggc gggagagccg tggcctgcac    1800
   ttcacagagg acttccctta cctggaggag agcaagagga agcctacagt gatcttccct    1860
   actgctatcc aagagctgac atggagttca aagccattgc agaggcagct gcagtgcaaa    1920
   tag                                                                  1923